# EUROPEAN PATENT SPECIFICATION

(11) **EP 2 934 582 B1**
(45) Date of publication and mention of the grant of the patent: **27.11.2019**
(21) Application number: 13808043.7
(22) Date of filing: 18.12.2013
(51) Int. Cl.: A61K 39/395, C07K 16/32

(54) **ANTI HER2 ANTIBODY FORMULATION**
ANTI-HER2-ANTIKÖRPERFORMULIERUNG
PRÉPARATION D'ANTICORPS ANTI-HER2

(30) Priority: 21.12.2012 US 201261745293 P
(43) Date of publication of application: 28.10.2015
(73) Proprietor: Glenmark Pharmaceuticals S.A., 2300 La Chaux-de-Fonds (CH)
(72) Inventor: ALBANESE, Jonathan André, CH-2300 La Chaux-de-Fonds (CH); GIOVANNINI, Roberto Pier-Lorenzo, CH-2300 La Chaux-de-Fonds (CH); O'MAHONY, Kevin Niall, CH-2300 La Chaux-de-Fonds (CH)
(74) Representative: Thomas, Dean
(86) International application number: PCT/EP2013/077166
(87) International publication number: WO 2014/096051

(56) References cited:
- WO-A1-97/04801
- WO-A2-03/062375
- WO-A2-2006/044908
- WO-A2-2011/012637
- MÁRK BAROK ET AL: "Trastuzumab causes antibody-dependent cellular cytotoxicity mediated growth inhibition of submacroscopic JIMT-1 breast cancer xenografts despite intrinsic drug resistance", MOLECULAR CANCER THERAPEUTICS, AMERICAN ASSOCIATION OF CANCER RESEARCH, US, vol. 6, no. 7, 1 July 2007 (2007-07-01), pages 2065-2072, XP008149732, ISSN: 1535-7163, DOI: 10.1158/1535-7163.MCT-06-0766
- DAUGHERTY A L ET AL: "Formulation and delivery issues for monoclonal antibody therapeutics", ADVANCED DRUG DELIVERY REVIEWS, ELSEVIER, AMSTERDAM, NL, vol. 58, no. 5-6, 7 August 2006 (2006-08-07), pages 686-706, XP024892149, ISSN: 0169-409X, DOI: 10.1016/J.ADDR.2006.03.011 [retrieved on 2006-08-07]
- MEYER J D ET AL: "Impact of bulking agents on the stability of a lyophilized monoclonal antibody", EUROPEAN JOURNAL OF PHARMACEUTICAL SCIENCES, ELSEVIER, AMSTERDAM, NL, vol. 38, no. 1, 12 August 2009 (2009-08-12), pages 29-38, XP026321300, ISSN: 0928-0987, DOI: 10.1016/J.EJPS.2009.05.008 [retrieved on 2009-05-23]
- NICHOLAS W WARNE ED - LEHR CLAUS-MICHAEL ET AL: "Development of high concentration protein biopharmaceuticals: The use of platform approaches in formulation development", EUROPEAN JOURNAL OF PHARMACEUTICS AND BIOPHARMACEUTICS, ELSEVIER SCIENCE PUBLISHERS B.V., AMSTERDAM, NL, vol. 78, no. 2, 3 March 2011 (2011-03-03), pages 208-212, XP028203394, ISSN: 0939-6411, DOI: 10.1016/J.EJPB.2011.03.004 [retrieved on 2011-03-13]

## Description

### Field of the Invention

The invention relates to a pharmaceutical formulation comprising an anti-HER2 antibody. Preferably, the pharmaceutical formulation comprises a lyophilized anti-HER2 antibody formulation. More preferably the formulation comprises a stable lyophilized anti-HER2 antibody formulation that has been reconstituted with a diluent to generate a stable reconstituted formulation.

### Background of the Invention

The ErbB receptor family is composed of four plasma membrane-bound receptor tyrosine kinases: EGFR/ErbB-1, HER2/ErbB-2, HER3/ErbB-3, and HER4/ErbB-4. Both homo- and heterodimers are formed by the four members of the EGFR family, with HER2 being the preferred and most potent dimerization partner for other ErbB receptors (Graus-Porta D et al., (1997) Embo J, 16: 1647- 1655; Tao RH et al., (2008) J Cell Sci, 121: 3207-3217). All four receptors contain an extracellular ligand binding domain, a transmembrane domain, and an intracellular domain that can interact with a multitude of signalling molecules and exhibit both ligand-dependent and ligand-independent activity. HER2 (Human Epidermal Growth Factor Receptor 2) also known as Neu, ErbB-2, CD340 (cluster of differentiation 340) or p185 is a protein that in humans is encoded by the *ERBB2* gene. HER2 activation leads to receptor phosphorylation, which triggers a cascade of downstream signals through multiple signalling pathways, such as MAPK, phosphoinositol 3-kinase/AKT, JAK/STAT and PKC, which ultimately results in the regulation of multiple cellular functions, such as growth, survival and differentiation (Huang Z et al., (2009) Expert Opin Biol Ther, 9: 97- 110). Over-expression of this gene has been shown to play an important role in the pathogenesis and progression of certain aggressive types of breast cancer.

Amplification or over-expression of the *ERBB2* gene occurs in approximately 30% of breast cancers. It is strongly associated with increased disease recurrence and a worse prognosis (Roy V & Perez EA (2009) Oncologist, 14(11): 1061-9). Over-expression is also associated with other human cancer types including: prostate cancer, non-small cell lung cancer, bladder cancer, ovarian cancer, gastric cancer, colon cancer, oesophageal cancer, squamous cell carcinoma of the head and neck and aggressive forms of uterine cancer, such as uterine serous endometrial carcinoma (Garcia de Palazzo I et al., (1993) Int J Biol Markers, 8: 233-239; Ross JS et al., (2003) Oncologist, 8: 307-325; Osman I et al., (2005) J Urol, 174: 2174-2177; Kapitanovic S et al., (1997) Gastroenterology, 112: 1103-1113; Turken O et al., (2003) Neoplasma, 50: 257-261; Oshima CT et al., (2001) Int J Biol Markers, 16: 250-254; Santin AD et al (2008) Int J Gynaecol Obstet, 102(2): 128-31).

Known drugs that target HER2 include the monoclonal antibody trastuzumab (Herceptin®), which is effective in cancers over expressing HER2. Another monoclonal antibody pertuzumab (Perjeta®/Omnitarg™) inhibits dimerization of HER2 and HER3 receptors. Different formulations have been proposed to stabilise these anti-HER2 antibodies. WO2003062375 reports a formulation for stabilising trastuzumab using glycine. In WO2011012637, a liquid formulation for subcutaneous injection of anti-HER2 antibodies, including Trastuzumab and Pertuzumab, has been reported. WO2006044908 describes a histidine-acetate buffered liquid formulation comprising Pertuzumab; while in WO1997004801 a stable lyophilized protein formulation, buffered with histidine or succinate, is proposed for proteins including anti-HER2 antibody.

The problem to be solved by the present invention is to provide a formulation for stabilising anti-HER2 antibody in a liquid formulation that can be used as base for the development of a lypophilized and a reconstituted formulations.

### Summary of the Invention

According to the invention there is provided a pharmaceutical formulation comprising an anti-HER2 antibody or a fragment thereof that recognizes and binds to human HER2 and which has a heavy chain of SEQ ID NO: 10 and a light chain of SEQ ID NO: 11; an acetate buffer, wherein the acetate buffer is sodium acetate buffer present within the pharmaceutical formulation in an amount of between 1 and 10mM; a surfactant, wherein the surfactant is Polysorbate 80, present within said pharmaceutical formulation in an amount of between 0.001%and 0.1% (w/w); a lyoprotectant, wherein the lyoprotectant is sucrose present within the pharmaceutical formulation in an amount of between 5% and 15% (w/w) or sucrose and mannitol present within the pharmaceutical formulation in an amount of 5% and 15% (w/w) and between 0.5% and 5% (w/w); and a bulking agent, wherein the bulking agent is glycine present within the pharmaceutical formulation in an amount of between 0.5% and 1.5%(w/w).

Therefore the invention also relates to a lyophilized formulation comprising an anti-HER2 antibody or a fragment thereof, a lyoprotectant, a bulking agent, a surfactant and an acetate buffer, wherein the pH of the formulation is pH 5.0-6.0, and its reconstituted formulation.

### Brief Description of the Figures

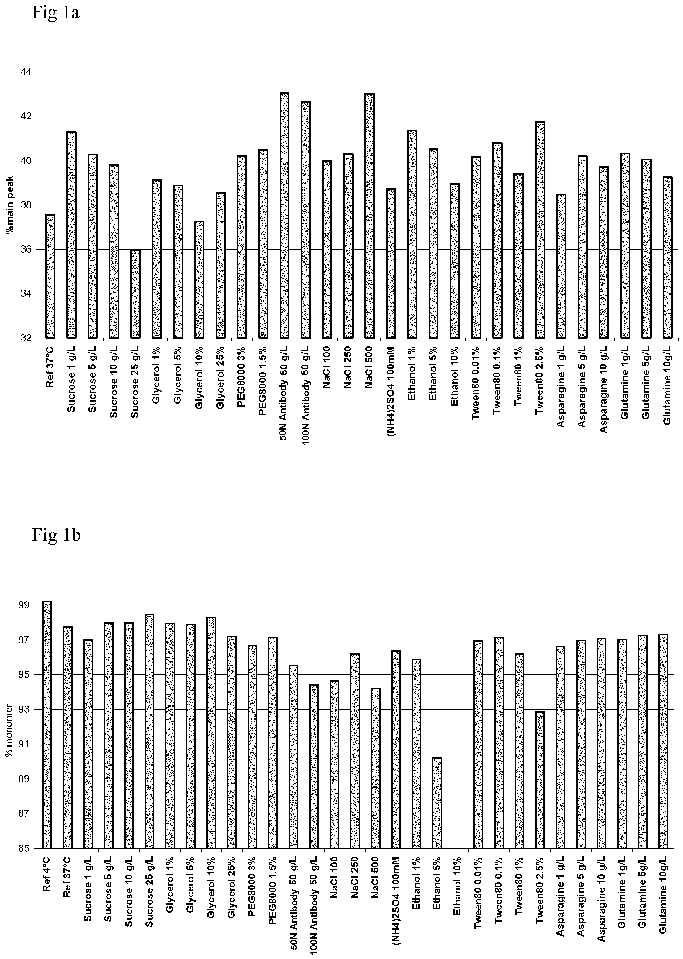

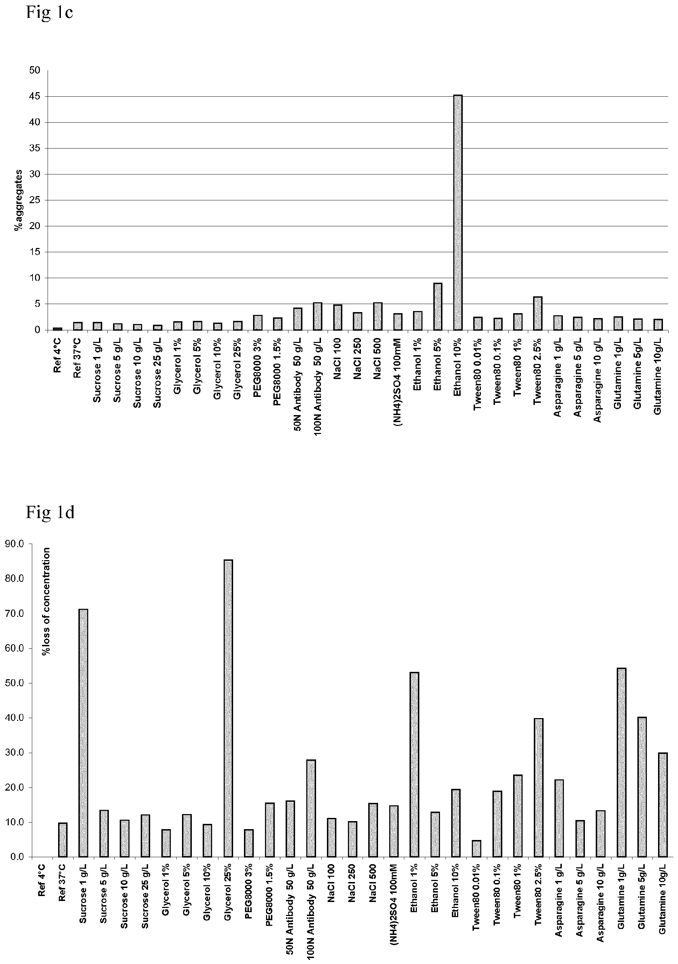

Figure 1 shows the results of an accelerated stability study of an anti-HER2 antibody at 37°C. The anti-HER2 antibody was formulated with various excipients that were verified for their potential in stabilising the antibody in a liquid form. The main peak percentage results by HP-CEX are shown in Figure 1a, the percentage of monomers by HP-SEC in Figure 1b, the percentage of aggregation in Figure 1c and the percentage loss of anti-HER2 antibody concentration in Figure 1d.

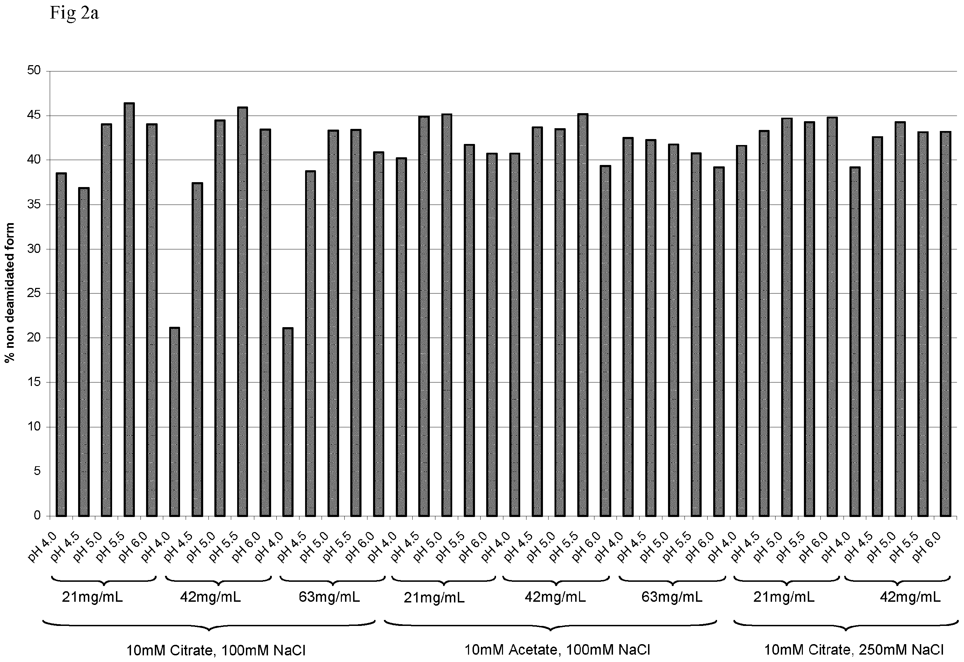

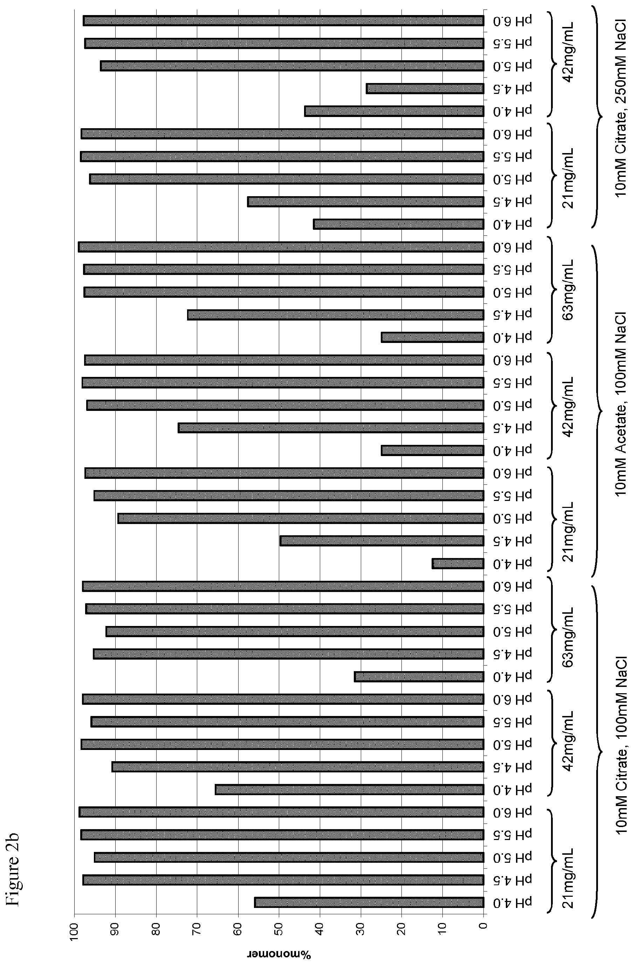

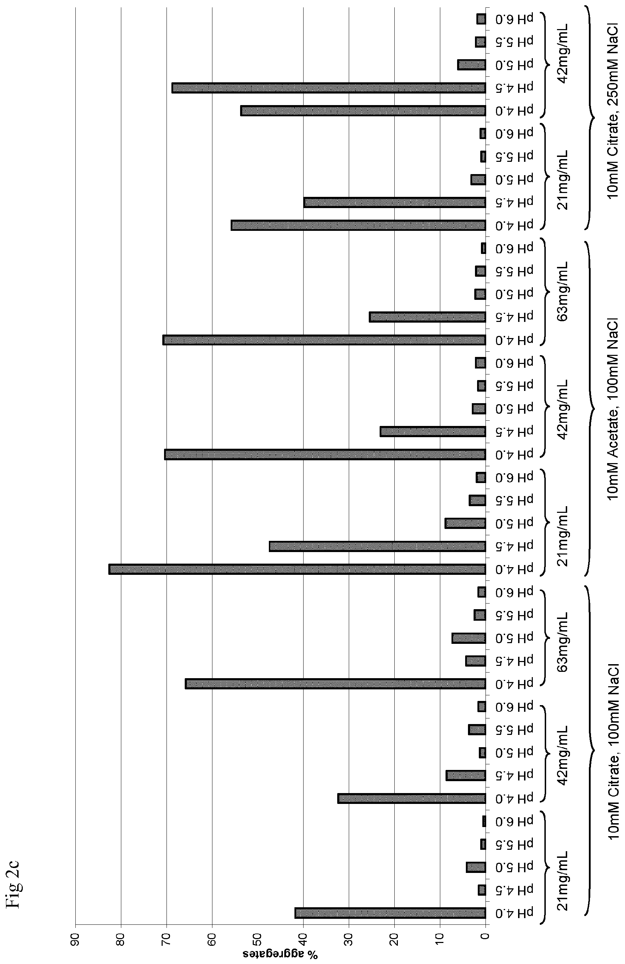

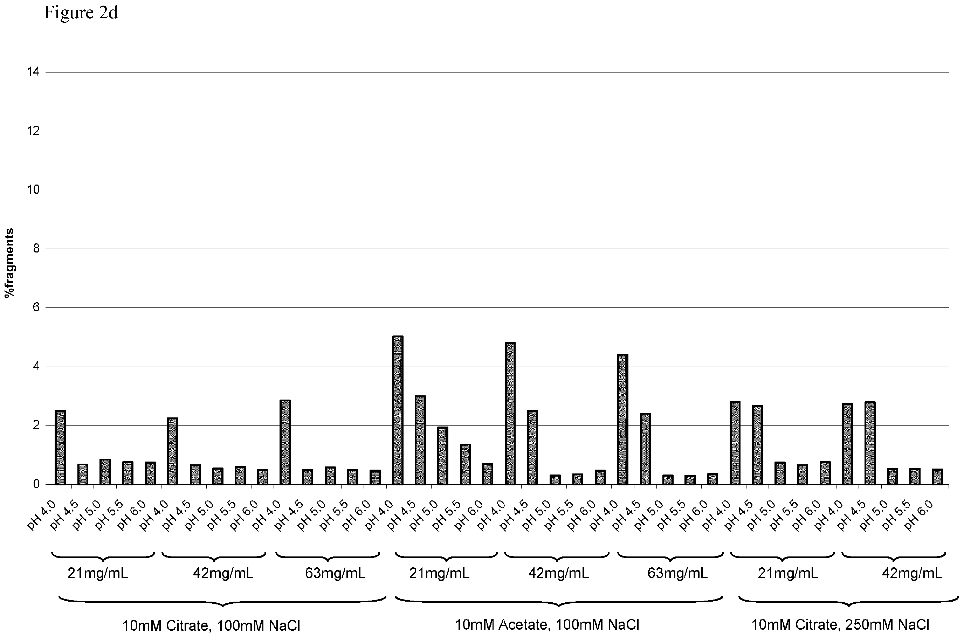

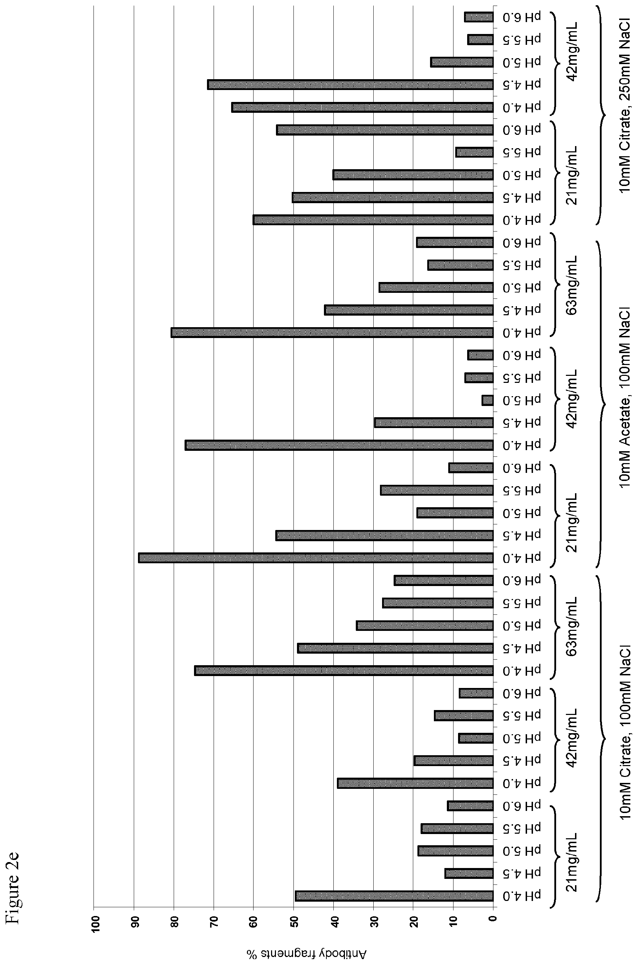

Figure 2 shows the results of an accelerated stability study of an anti-HER2 antibody at 37°C, in a liquid formulation. Different concentrations of the anti-HER2 antibody were formulated at pH values in the range of pH 4.0 to pH 6.0 and with different buffers, either citrate or acetate. The main peak by HP-CEX results are shown in Figure 2a, the percentage of monomers by HP-SEC in Figure 2b, the percentage of aggregation in Figure 2c, the percentage of fragmentation in Figure 2d and the percentage loss of anti-HER2 antibody concentration in Figure 2e.

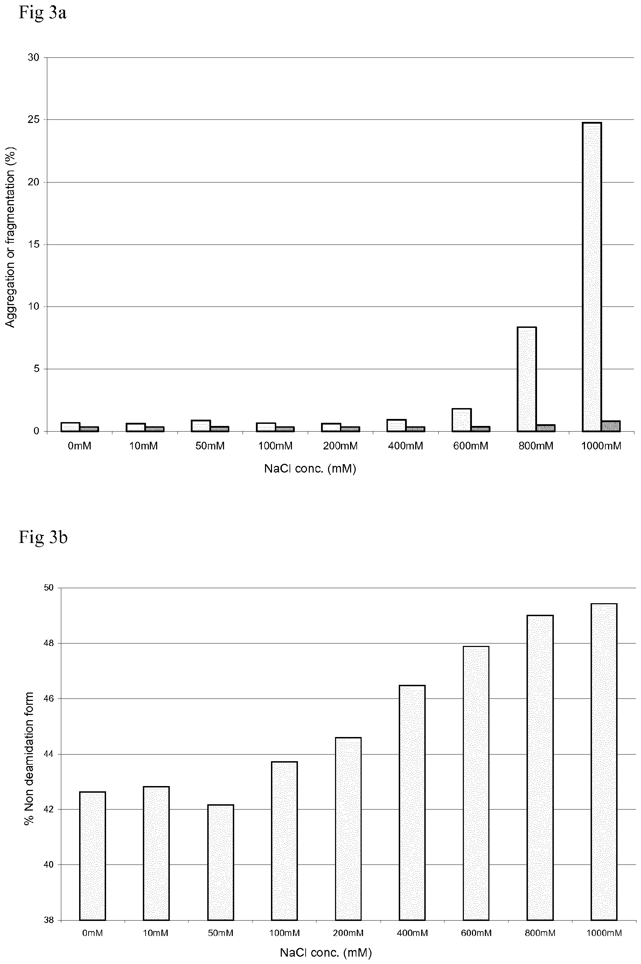

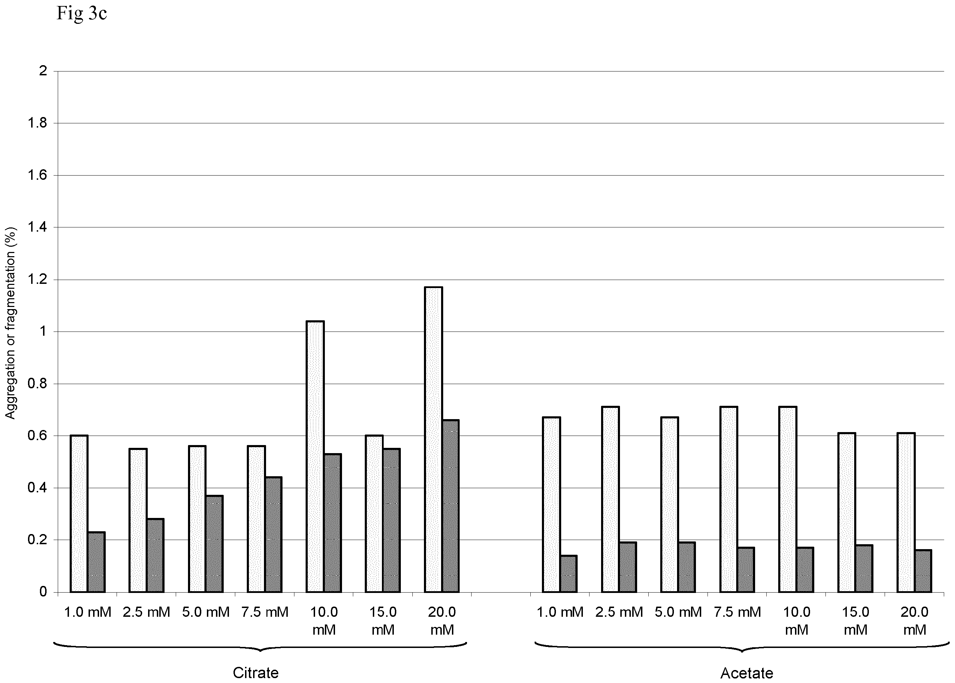

Figure 3 shows the results of a screen to evaluate the optimal ranges of salt concentration, buffer type and antioxidant for the anti-HER2 antibody liquid formulation kept for one month at 37°C. Figure 3a shows the aggregation and fragmentation at various NaCl concentrations at pH 5.5. The y-axis shows percentage aggregation or fragmentation. Aggregation is shown in the light grey bars, left hand side and fragmentation is shown in the dark grey bars, right hand side; for each concentration of NaCl. Figure 3b shows anti-HER2 antibody deamidation at various NaCl concentrations at pH 5.5. Figure 3c shows the percentage of aggregation and fragmentation of the anti-HER2 antibody when formulated with citrate or acetate at various concentrations at pH 5.5. Aggregation is shown in the light grey bars, left hand side and fragmentation is shown in the dark grey bars, right hand side; for each concentration of citrate or acetate.

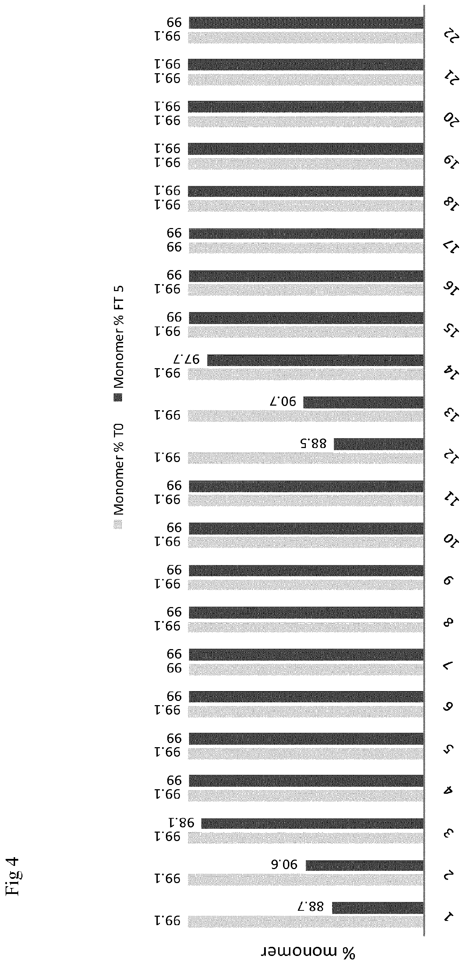

Figure 4 shows the percentage of monomers present in the 22 formulations tested (see Table 8 for details) at time 0 (light coloured bars; left-hand side) and after five cycles of freeze-thaw (dark coloured bars; right-hand side). Each formulation tested is shown on the x-axis with the percentage of monomer shown on the y-axis. Formulations 6, 8 10 and 19 were selected for further experimental work.

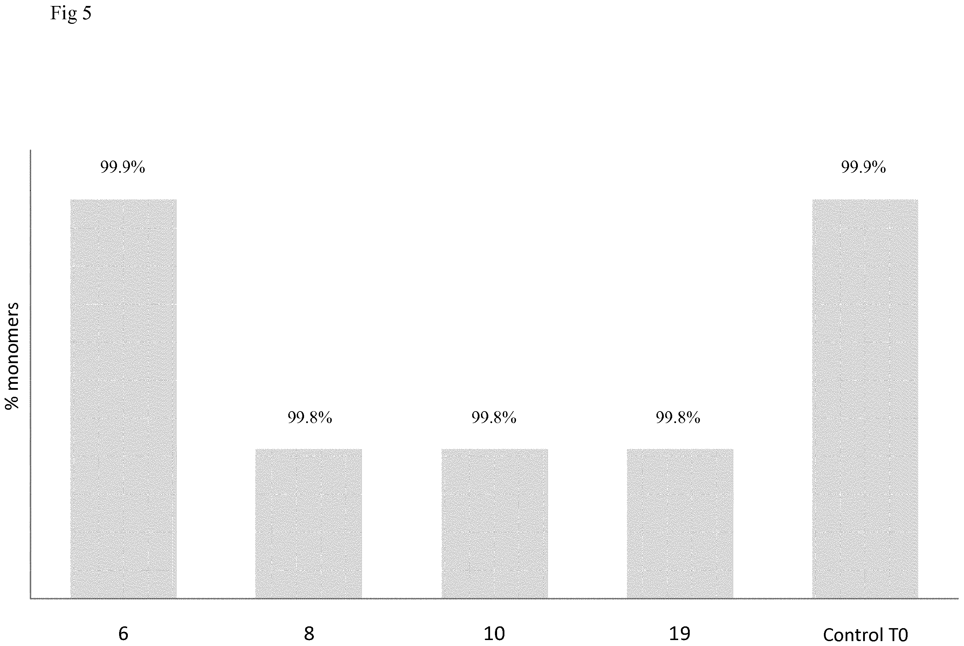

Figure 5 shows the percentage of monomers present in the four formulations tested after reconstitution of the cakes. All amounts are shown as % monomers for formulations 6, 8, 10 and 19 selected from the previous screen. The column on the far right is a control at time 0.

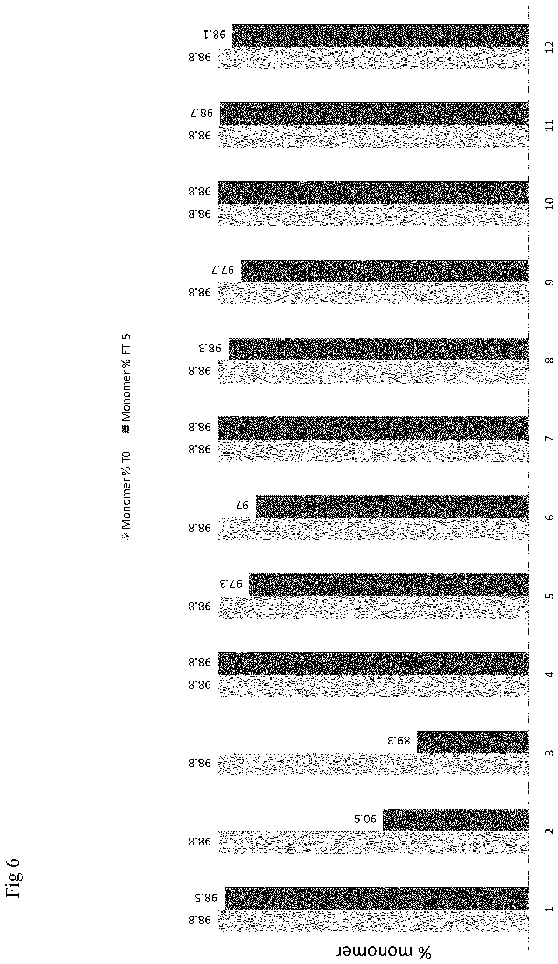

Figure 6 shows the percentage of monomers present in the 12 formulations tested (see Table 11 for details) at time 0 (light coloured bars; left-hand side) and after five cycles of freeze-thaw (dark coloured bars; right-hand side). Each formulation tested is shown on the x-axis with the percentage of monomer shown on the y-axis. Formulations 1, 4, 7, 10 and 11 were selected for lyophilization.

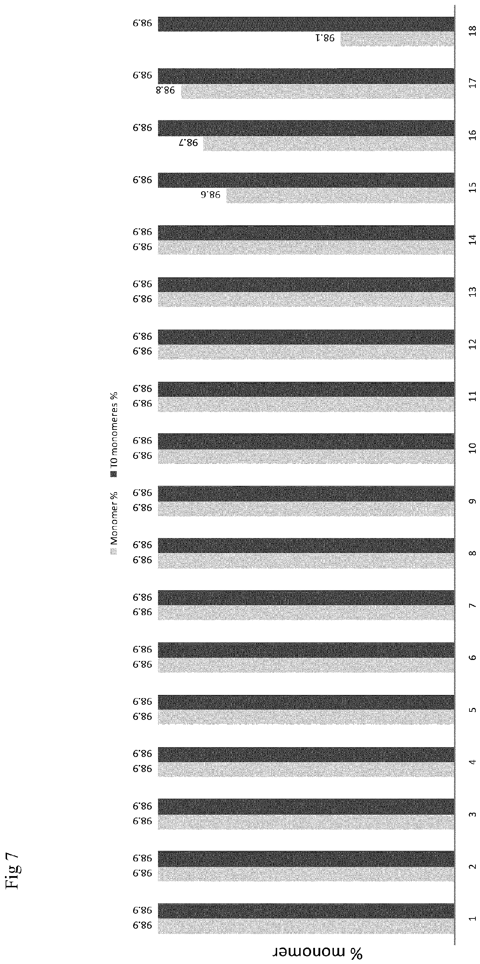

Figure 7 shows the percentage of monomers present in 18 formulations tested (see Table 12 for details) at time 0 (light coloured bars; left-hand side) and after lyophilization and high pressure liquid chromatography - size exclusion (dark coloured bars; right-hand side). Each formulation tested is shown on the x-axis with the percentage of monomer shown on the y-axis.

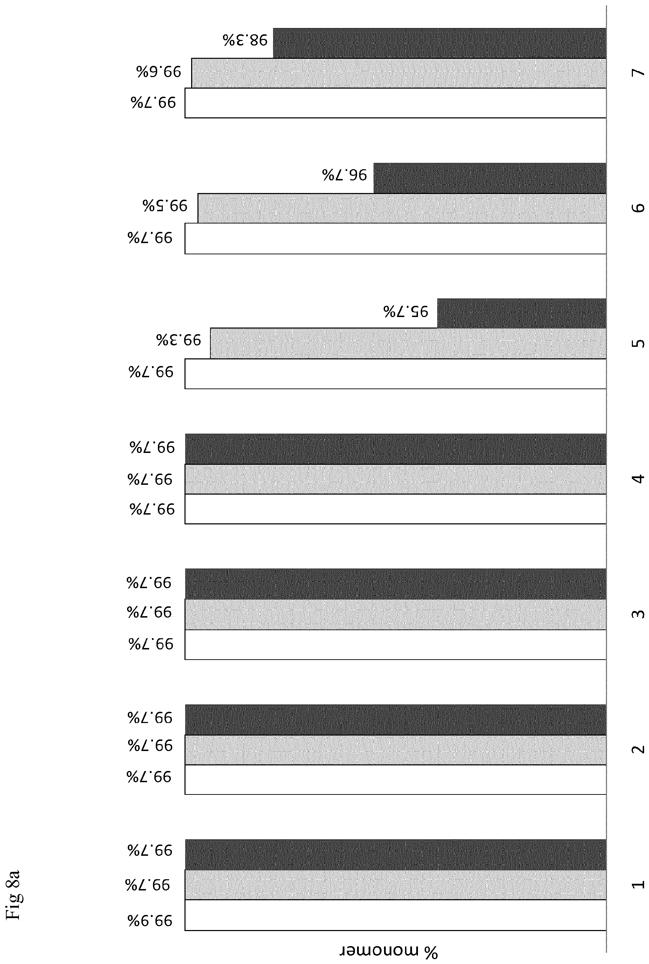

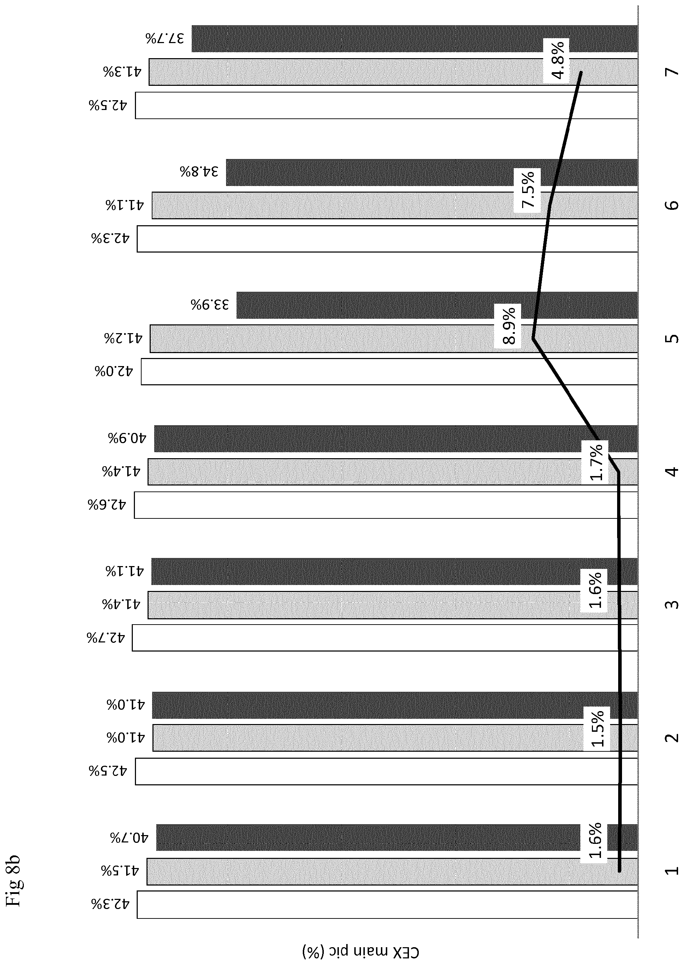

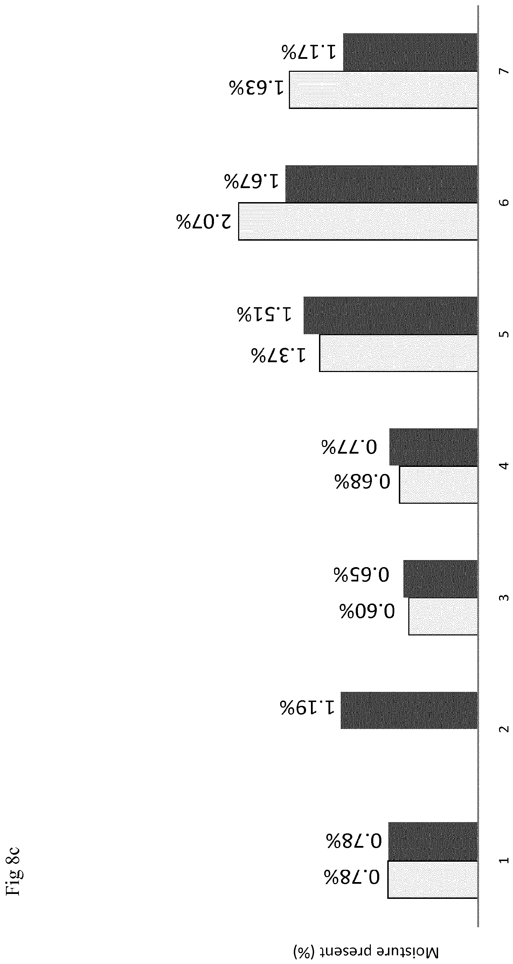

Figure 8 shows the stability results for seven lyophilized formulations tested (see Table 13 for details) in a one month stability study at 40°C. Figure 8a shows the percentage of monomers present in the formulations tested at time 0 (white bars; left-hand side), after lyophilization (light coloured bars; centre) and after one month at 40°C (dark coloured bars; right-hand side). Each formulation tested is shown on the x-axis with the percentage of monomer shown on the y-axis. Figure 8b shows the % main peak after cation exchange chromatography for the formulations tested at time 0 (white bars; left-hand side), after lyophilization (light coloured bars; centre) and after one month at 40°C (dark coloured bars; right-hand side). The line connecting the columns indicates the percentage loss of main peak for each formulation after one month storage at 40°C. Figure 8c shows the percentage of moisture present for each formulation, tested after lyophilization (light coloured bars; left-hand side) and after storage for one month at 40°C (dark coloured bars; right-hand side).

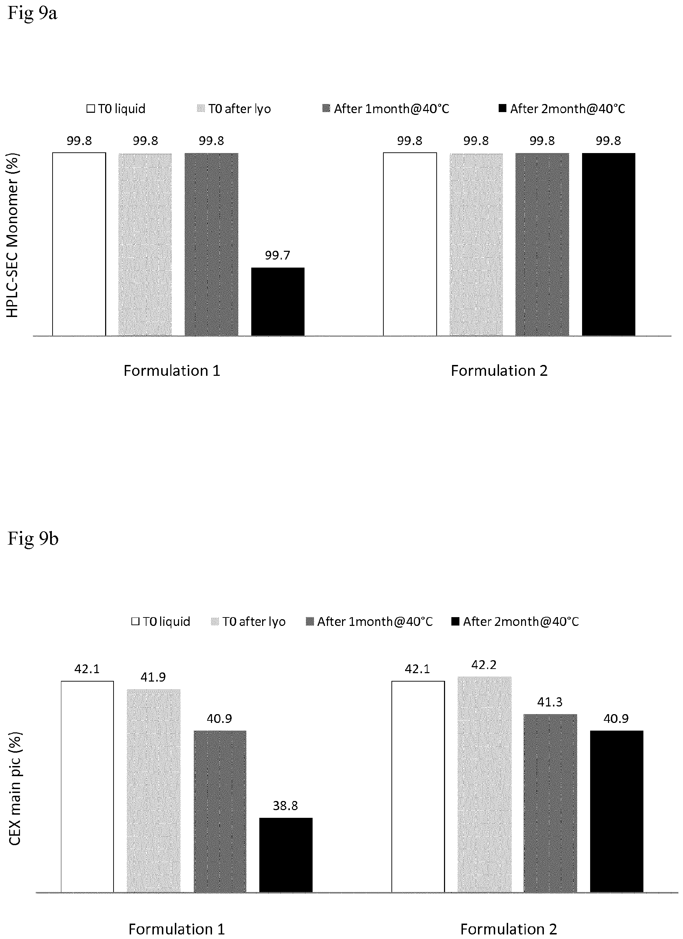

Figure 9 shows the stability results for Formulations 1 and 2 (see Table 16 for details) in an accelerated stability study for the time periods time 0 (liquid and lyophilized formulations; clear and light grey bars respectively), one month at 40°C (dark gray bars) and two months at 40°C (black bars). Figure 9a shows the percentage monomers (y-axis) present in Formulations 1 and 2 as measured by HPLC-SEC and Figure 9b shows the HPLC-CEX percentage main peak (y-axis) for Formulations 1 and 2.

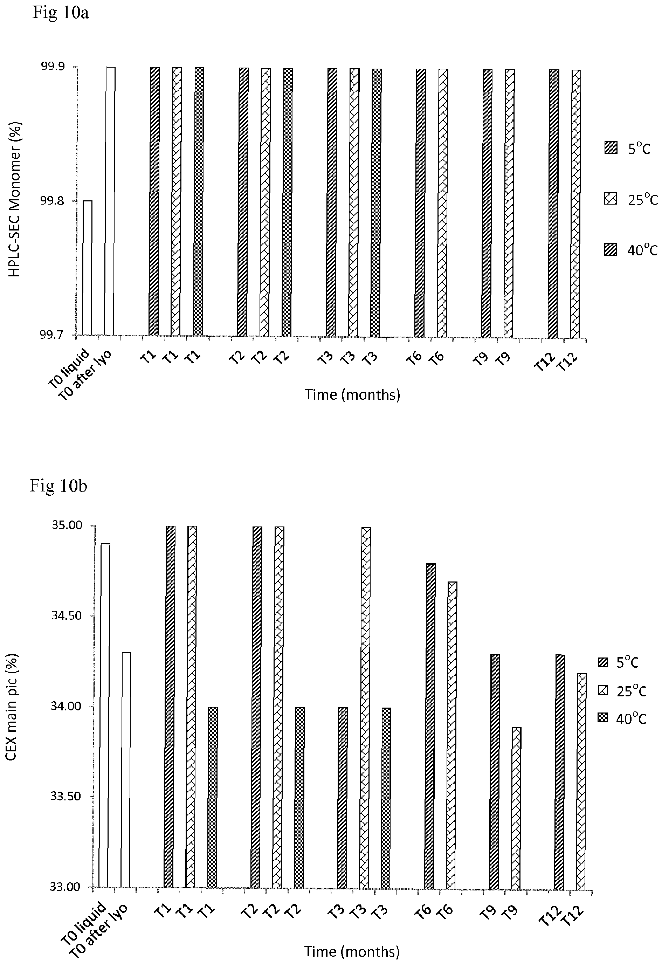

Figure 10 shows the stability results for the lyophilized anti-HER2 antibody formulation in a long term stability study for the time periods indicated on the x-axis. Figure 10a shows the percentage monomer (y-axis) present in the formulation as measured by HPLC-SEC at the time periods of 1, 2, 3, 6, 9 and 12 months. Figure 10b shows the HPLC-CEX percentage main pic (y-axis) for the formulation at the time periods of 1, 2, 3, 6, 9 and 12 months.

### Detailed Description of the Invention

The present disclosure related to a pharmaceutical formulation comprising an antibody or a fragment thereof that recognises and binds to human HER2.

The term "human HER2" as used herein includes variants, isoforms, and species homologs of human HER2. Accordingly, antibodies of the invention may, in certain cases, cross-react with HER2 from species other than human. In certain embodiments, the antibodies may be completely specific for one or more human HER2 proteins and may not exhibit species or other types of non-human cross-reactivity. The complete amino acid sequence of an exemplary human HER2 has Swiss-Prot accession number P04626 (ERBB2_HUMAN; SEQ ID NO: 1). HER2 is also known as CD340, MLN 19, Neu, c-ErbB-2 and p185erbB2. Human HER2 is designated GeneID: 2064 by Entrez Gene, and HGNC: 3430 by HGNC. HER2 can be encoded by the gene designated *ERBB2.*

The use of "HER2" herein encompasses all known or as yet undiscovered alleles and polymorphic forms of human HER2. The terms "human HER2" or "HER2" are used herein equivalently and mean "human HER2" if not otherwise specifically indicated.

The terms "antibody that binds to HER2" and "anti-HER2 antibody" are used herein interchangeably and include antibodies or a fragment thereof that bind to human HER2 e.g. human HER2 in isolated form.

In a highly preferred embodiment, the anti-HER2 antibody comprises a heavy chain and light chain of SEQ ID NO: 10 and SEQ ID NO: 11.

As used herein, the term "HER2-related disorder" includes conditions such as cancer, particularly metastatic breast cancer, early breast cancer and metastatic gastric cancer and more particularly HER2-positive metastatic breast cancer.

As used herein, the term "subject" includes any human or non-human animal. The term "non-human animal" includes all vertebrates, e.g., mammals and non-mammals, such as non-human primates, sheep, dogs, cats, horses, cows, chickens, amphibians, reptiles, etc. Preferably the subject is human.

A "stable" formulation is one in which the protein therein essentially retains its physical stability and/or chemical stability and/or biological activity upon storage. Various analytical techniques for measuring protein stability are available in the art and are reviewed for example in Peptide and Protein Drug Delivery, 247-301, Vincent Lee Ed., Marcel Dekker, Inc., New York, New York, Pubs. (1991) and Jones A (1993) Adv Drug Delivery Rev, 10: 29-90. Stability can be measured at a selected temperature for a selected time period. Preferably, the formulation is stable at room temperature (25°C) or at 40°C for at least 1 month, preferably 2 months, more preferably 6 months and/or stable at about 5°C for at least 1 year and preferably for at least 2 years. Furthermore, the formulation is preferably stable following freezing (to for example, -40°C) and thawing of the formulation.

The "physical stability" of a protein in a pharmaceutical formulation is retained if it shows no signs of aggregation, precipitation and/or denaturation upon visual examination of colour and/or clarity, or as measured by UV light scattering or by size exclusion chromatography.

The "chemical stability" of a protein can be assessed by detecting and quantifying chemically altered forms of the protein. Chemical alteration may involve size modification (e.g. clipping) which can be evaluated using size exclusion chromatography, SDS-PAGE and/or matrix assisted laser desorption ionization/time-of-flight mass spectrometry (MALDI/TOF MS), for example. Other types of chemical alteration include charge alteration (e.g. occurring as a result of deamidation) which can be evaluated by ion exchange chromatography, for example.

The term "buffer" refers to a buffered solution that resists changes in pH by the action of its acid-base conjugate components. A buffer of this invention has a pH in the range from about 5.0 to about 6.0; and preferably 5.7. Examples of buffers that can control the pH in this range include acetate (e.g. sodium acetate), succinate (such as sodium succinate), gluconate, histidine, citrate and other organic acid buffers.

A "lyoprotectant" is a molecule which when combined with a protein of interest significantly prevents or reduces chemical and/or physical instability of the protein upon lyophilization and subsequence storage. Examples of lyoprotectants include a polyol, an amino acid, a methylamine such as betaine, a lyotropic salt such as magnesium sulphate, propylene glycol, polyethylene glycol, Pluronics, and combinations thereof. The preferred lyoprotectant is a non-reducing sugar such as sucrose and/or a sugar alcohol such as mannitol, more preferably a non-reducing sugar such as sucrose. The lyoprotectant is added to the pre-lyophilisation formulation in a "lyoprotecting amount" which means that following lyophilisation of the protein in the presence of the lyoprotecting amount of the lyoprotectant, the protein retains its physical and chemical stability and integrity upon lyophilization and storage.

A "polyol" is a substance with multiple hydroxyl groups, and includes sugars (reducing and non-reducing sugars), sugar alcohols and sugar acids. Preferred polyols herein have a molecular weight which is less than about 600kD (e.g. in the range from about 120 to about 400 kD). A "reducing sugar" is one which contains a hemiacetal group that can reduce metal ions or react covalently with lysine and other amino groups in proteins and a "non-reducing sugar" is one which does not have these properties of a reducing sugar. Examples of reducing sugars are fructose, mannose, maltose, lactose, arabinose, xylose, ribose, rhamnose, galactose and glucose. Non-reducing sugars include sucrose, trehalose, sorbose and raffinose. Sugar alcohols include mannitol, xylitol, erythritol, threitol, sorbitol and glycerol. A polyol for use in a formulation that is freeze-thaw stable is one which does not crystallize at freezing temperatures (e.g.-20°C) such that it destabilises the antibody in the formulation.

A "bulking agent" is a compound which adds mass to the lyophilized mixture and contributes to the physical structure of the lyophilized cake (e.g. facilitates the production of an essentially uniform lyophilized cake which maintains an open pore structure) Examples of bulking agents include mannitol, glycine, polyethylene glycol and xorbitol.

A "liquid" formulation is one that has been prepared in a liquid format. Such a formulation may be suitable for direct administration to a subject or, alternatively, can be packaged for storage either in a liquid form, in a frozen state or in a dried form (e.g. lyophilized) for later reconstitution into a liquid form or other form suitable for administration to a subject.

A "lyophilized" formulation is one that has been prepared by freeze-drying a liquid or pre-lyophilization formulation. Freeze-drying is performed by freezing the formulation and then subliming ice from the frozen content at a temperature suitable for primary drying. Under this condition the product temperature is below the collapse temperature of the formulation. A secondary drying stage may then be carried out, which produces a suitable lyophilized cake.

A "reconstituted" formulation is one that has been prepared by dissolving a lyophilized protein formulation in a diluent such that the protein is dispersed in the reconstituted formulation. The reconstituted formulation should be suitable for administration (e.g. parenteral administration) to a subject to be treated with the protein of interest. Suitable "diluents" useful for the preparation of a reconstituted formulation include ones which are pharmaceutically acceptable (safe and non-toxic for administration to a human). Examples of suitable diluents include sterile water, bacteriostatic water for injection (BWFI). Water for injection (WFI), a pH buffered solution e.g. phosphate-buffered saline (PBS), sterile saline solution, Ringer's solution or dextrose solution.

The antibody comprised by the formulation of the invention can be produced e.g. by recombinant technology. A nucleic acid encoding a HER2 antibody of the invention (e.g. CDR or set of CDRs or VH domain or VL domain or antibody antigen-binding site or antibody molecule, e.g. scFv or IgG1 as provided), may be expressed by culturing under appropriate conditions recombinant host cells containing said nucleic acid. Following production by expression a VH and/or VL domain, for example, may be isolated and/or purified using any suitable technique, then used as appropriate.

Antibodies, VH and/or VL domains, and encoding nucleic acid molecules and vectors may be provided isolated and/or purified, e.g. from their natural environment, in substantially pure or homogeneous form, or, in the case of nucleic acid, free or substantially free of nucleic acid or gene origin other than the sequence encoding a polypeptide with the required function. Nucleic acid may comprise DNA or RNA and may be wholly or partially synthetic.

Systems for cloning and expression of a polypeptide in a variety of different host cells are well known. Suitable host cells include bacteria, mammalian cells, plant cells, yeast and baculovirus systems and transgenic plants and animals.

Mammalian cell lines available in the art for expression of a heterologous polypeptide include Chinese hamster ovary (CHO) cells, HeLa cells, baby hamster kidney cells, NSO mouse melanoma cells, YB2/0 rat myeloma cells, human embryonic kidney cells, human embryonic retina cells and many others.

Preferably, the mammalian cell line is Chinese hamster ovary (CHO) cells. These may be dihydrofolate reductase (dhfr) deficient and so dependent on thymidine and hypoxanthine for growth (Urlaub G & Chasin LA (1980) PNAS, 77: 4216- 4220). The parental dhfr CHO cell line is transfected with the antibody gene and dhfr gene which enables selection of CHO cell transformants of dhfr positive phenotype. Selection is carried out by culturing the colonies on media devoid of thymidine and hypoxanthine, the absence of which prevents untransformed cells from growing and transformed cells from resalvaging the folate pathway and thereby bypassing the selection system. These transformants usually express low levels of the product gene by virtue of co- integration of both transfected genes. The expression levels of the antibody gene may be increased by amplification using methotrexate (MTX). This drug is a direct inhibitor of the dhfr enzyme and allows isolation of resistant colonies which amplify their dhfr gene copy number sufficiently to survive under these conditions. Since the dhfr and antibody genes are more closely linked in the original transformants, there is usually concomitant amplification, and therefore increased expression of the desired antibody gene. Another selection system for use with CHO or myeloma cells is the glutamine synthetase (GS) amplification system described in WO87/04462. This system involves the transfection of a cell with a gene encoding the GS enzyme and the desired antibody gene. Cells are then selected which grow in glutamine free medium. These selected clones are then subjected to inhibition of the GS enzyme using methionine sulphoximine (MSX). The cells, in order to survive, will amplify the GS gene with concomitant amplification of the gene encoding the antibody of interest.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 3rd edition, Sambrook and Russell, 2001, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1988, Short Protocols in Molecular Biology; A Compendium of Methods from Current Protocols in Molecular Biology, Ausubel et al. eds. , John Wiley & Sons, 4th edition 1999.

Introduction of a nucleic acid into a host cell may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. Introducing nucleic acid in the host cell, in particular a eukaryotic cell may use a viral or a plasmid based system. The plasmid system may be maintained episomally or may be incorporated into the host cell or into an artificial chromosome (Csonka E et al., (2000) Journal of Cell Science, 113: 3207-3216; Vanderbyl S et al., (2002) Molecular Therapy, 5(5): 10. Incorporation may be either by random or targeted integration of one or more copies at single or multiple loci. For bacterial cells, suitable techniques may include calcium chloride transformation, electroporation and infection using bacteriophage.

The introduction may be followed by causing or allowing expression from the nucleic acid, e.g. by culturing host cells under conditions for expression of the gene. In one embodiment, the nucleic acid of the invention is integrated into the genome (e. g. chromosome) of the host cell. Integration may be promoted by inclusion of sequences which promote recombination with the genome, in accordance with standard techniques.

A further embodiment provides a process for purifying a HER2 antibody which comprises one or more chromatographic separation steps wherein each of said separation steps comprises elution with an elution buffer comprising one or more pharmaceutically acceptable excipients.

Preferably, the one or more chromatographic separation steps are selected from affinity chromatography (e.g. Protein A or Protein G affinity chromatography), ion exchange chromatography (e.g. cation and anion exchange chromatography), hydrophobic interaction chromatography (e.g. phenyl chromatography), hydroxy apatite chromatography, size exclusion chromatography, immobilised metal affinity chromatography, hydrophilic interaction chromatography, thiophilic adsorption chromatography, euglobulin adsorption chromatography, dye ligand chromatography or immobilised boronate chromatography. Most preferably, chromatographic separation is performed by Protein A affinity chromatography followed by cation exchange chromatography and/or followed by hydrophobic interaction chromatography or anion exchange chromatography.

### Pharmaceutical Formulations

The pharmaceutical formulation of the invention may be a liquid formulation, a lyophilized formulation or a reconstituted formulation.

The desired dose volume and ultimate mode of administration of the formulation is taken into account when determining the amount of anti-HER2 antibody or a fragment thereof to include in the formulation. Preferably the anti-HER2 antibody of a fragment thereof is present within the pharmaceutical formulation in an amount of between 1mg/ml and 100mg/ml, more preferably 5mg/ml and 50mg/ml, even more preferably 10mg/ml and 40mg/ml, especially 20mg/ml and 30mg/ml.

The formulation can be buffered to a pH of 5.0 - 6.0, preferably pH 5.5 - 5.9, more preferably pH 5.6 - 5.8, even more preferably pH 5.7 ± 0.2, most preferably pH 5.7 ± 0.1. The specification provides buffers that can be used to control the pH in this range selected from the group consisting of acetate, citrate, succinate, gluconate, histidine, phosphate, glutaric, cacodylyte, sodium hydrogen maleate, tris- (hydroxylmethyl) aminomethane (Tris), 2-(N-morpholino) ethanesulphonic acid (MES), imidazole and other organic acid buffers. Preferably, the buffer is acetate buffer, more preferably sodium acetate. Preferably, the acetate buffer is present within the formulation in an amount of between 1-50mM, more preferably 1-20mM, even more preferably 1-10mM.

Preferably, a "pharmaceutically acceptable excipient" is added to the liquid formulation. It will be appreciated that references to "pharmaceutically acceptable excipient" includes references to any excipient conventionally used in pharmaceutical formulations. Such excipients may typically include one or more surfactant, lyoprotectant, bulking agent, inorganic or organic salt, stabilizer, diluent, solubilizer, reducing agent, antioxidant, chelating agent, preservative and the like.

Since it is desirable for a parenteral formulation to be isotonic with body fluids (i.e. approximately 284mOsm/L), the specification provides a formulation in which a tonicifier may be added to the pharmaceutical formulation. An example of a commonly used tonicifier is sodium chloride salt. Preferably, the tonicifier is present in the liquid formulation at a concentration of 50 to 400mM, more preferably 100 to 300mM, even more preferably 200 to 250mM. However a tonicifier is not usually added to a lyophilized formulation, therefore prior to lyophilisation, a tonicifier may be removed from the formulation. A tonicifier may be present in the diluent for reconstituted formulations.

The specification provides a surfactant selected from the group consisting of: non-ionic surfactants (HLB 6 to 18) such as sorbitan fatty acid esters (e.g. sorbitan monocaprylate, sorbitan monolaurate, sorbitan monopalmitate), glycerine fatty acid esters (e.g. glycerine monocaprylate, glycerine monomyristate, glycerine monostearate), poly glycerine fatty acid esters (e.g. decaglyceryl monostearate, decaglyceryl distearate, decaglyceryl monolinoleate), polyoxyethylene sorbitan fatty acid esters (e.g. polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, polyoxyethylene sorbitan tristearate), polyoxyethylene sorbitol fatty acid esters (e.g. polyoxyethylene sorbitol tetrastearate, polyoxyethylene sorbitol tetraoleate), polyoxyethylene glycerine fatty acid esters (e.g. polyoxyethylene glyceryl monostearate), polyethylene glycol fatty acid esters (e.g. polyethylene glycol distearate), polyoxyethylene alkyl ethers (e.g. polyoxyethylene lauryl ether), polyoxyethylene polyoxypropylene alkyl ethers (e.g. polyoxyethylene polyoxypropylene glycol ether, polyoxyethylene polyoxypropylene propyl ether, polyoxyethylene polyoxypropylene cetyl ether), polyoxyethylene alkylphenyl ethers (e.g. polyoxyethylene nonylphenyl ether), polyoxyethylene hydrogenated castor oils (e.g. polyoxyethylene castor oil, polyoxyethylene hydrogenated castor oil), polyoxyethylene beeswax derivatives (e.g. polyoxyethylene sorbitol beeswax), polyoxyethylene lanolin derivatives (e.g. polyoxyethylene lanolin), and polyoxyethylene fatty acid amides (e.g. polyoxyethylene stearyl amide); anionic surfactants such as C₁₀-C₁₈ alkyl sulfates salts (e.g. sodium cetyl sulfate, sodium lauryl sulfate, sodium oleyl sulfate), polyoxyethylene C₁₀-C₁₈ alkyl ether sulfates salts with an average of 2 - 4 moles of ethylene oxide (e.g. sodium polyoxyethylene lauryl sulfate), and C₈-C₁₈ alkyl sulfosuccinate ester salts (e.g. sodium lauryl sulfosuccinate ester); and natural surfactants such as lecithin, glycerophospholipid, sphingophospholipids (e.g. sphingomyelin) and sucrose esters of C₁₂-C₁₈ fatty acids.

Preferably, the surfactant is selected from polyoxyethylene sorbitan fatty acid esters. Particularly preferably the surfactant is Polysorbate 20, 21, 40, 60, 65, 80, 81 and 85, most preferably Polysorbate 80. Polysorbate 80 is also known by the brand name Tween 80™ (ICI Americas, Inc.).
Preferably, the surfactant is present within the formulation in an amount of between 0.001 and 0.1% (w/w), more preferably between 0.001 and 0.05% (w/w), even more preferably between 0.005 and 0.02% (w/w).

A solubilizer may also be added to increase the solubilisation of the antibody in solution. Examples of solubilizers include amino acids such as proline or glycine, prophylene glycol, plasdone C and K povidones, cyclodextrins and plasdone K polymers. Preferably, the solubilizer is added to the formulation at a concentration of 1-50mg/ml, preferably 5-40mg/ml, more preferably 10-30mg/ml.

The specification provides a pharmaceutical formulations of the invention that may also comprise additional excipients such as reducing agents, antioxidants and/or chelating agents.

Examples of a reducing agent include N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione and a C₁-C₇ thioalkanoic acid.

Examples of an antioxidant include amino acids such as methionine, erythorbic acid, dibutylhydroxytoluene, butylhydroxyanisole, alpha-tocopherol, tocopherol acetate, L-ascorbic acid and a salt thereof, L-ascorbic acid palmitate, L-ascorbic acid stearate, sodium bisulfite, sodium sulfite, triamyl gallate and propyl gallate.

Examples of a chelating agent include disodium ethylenediaminetetraacetate (EDTA), sodium pyrophosphate and sodium metaphosphate.

Not all excipients in a liquid formulation are suitable for inclusion a lyophilized form of the formulation and therefore a number of changes may be made to the liquid formulation to make it suitable for freeze-drying.

A stabilizer may be added to the formulation to stabilise the protein in the lyophilised form. Examples of a stabiliser include creatinine, an amino acid selected from histidine, alanine, glutamic acid, glycine, leucine, phenylalanine, methionine, isoleucine, proline, aspartic acid, arginine, lysine and threonine, a carbohydrate selected from sucrose, trehalose, sorbitol, xylitol and mannose, surfactants selected from polyethylene glycol (PEG; e.g. PEG3350 or PEG4000) or polyoxyethylene sorbitan fatty acid esters (e.g. Polysorbate 20 or Polysorbate 80), or any combination thereof.

In a preferred aspect the stabiliser comprises a lyoprotectant, which may be selected from a non-reducing sugar (e.g. sucrose or trehalose) and/or a sugar alcohol (e.g. mannitol). Addition of a lyoprotectant helps to reduce the amount of degradation or aggregation of the protein upon lyophilisation. Preferably, the lyophilized formulation is isotonic upon reconstitution; therefore the amount of lyoprotectant in the lyophilized formulation should be sufficient to achieve an isotonic reconstituted formulation. Alternatively, the reconstituted formulation may be hypertonic and therefore a greater amount of lyoprotectant is required in the lyophilized formulation. Conversely, if too little lyoprotectant is added to the lyophilized formulation, then an unacceptable amount of antibody degradation or aggregation may occur upon lyophilisation.

Preferably, the lyoprotectant in the lyophilized formulation is a non-reducing sugar and/or a sugar alcohol, more preferably sucrose and/or mannitol. In preferred embodiment the lyoprotectant in the lyophilized formulation is sucrose. Preferably the lyoprotectant is present in the lyophilized formulation at a concentration from about 10mM to about 700mM, preferably from about 50mM to about 600mM, more preferably from about 100mM to about 500mM, even more preferably from about 200mM to about 400mM.

Preferably, the lyoprotectant is present in the lyophilized formulation in an amount of between 1-100% (w/w), preferably 2-50% (w/w), more preferably 3-25%, even more preferably 5-20% (w/w).

The specification provides a lyophilized formulation comprising a lyoprotectant in which preferably the molar ratio of antibody to lyoprotectant may be in the range from 100 to about 3000 moles lyoprotectant to 1 mole antibody, preferably from about 500 to about 2500 moles lyoprotectant to 1 mole antibody, more preferably from about 1000 to about 2250 moles lyoprotectant to 1 mole antibody, even more preferably from about 1500 to 2000 moles.

Preferably, the lyophilized formulation comprises a bulking agent. Examples of bulking agents include mannitol, glycine, polyethylene glycol, xorbitol, anhydrous lactose, sucrose, D(+)-trehalose, dextran 40 and povidone (PVP K24). Preferably, the bulking agent is present in the lyophilized formulation at a concentration from about 50 to 200mM, preferably from about 100 to 150mM. Preferably, the bulking agent is present in the lyophilized formulation in an amount of between 0.1 and 10% (w/w), preferably between 0.2 and 8% (w/w), more preferably between 0.5 and 5% (w/w).

Once a lyophilized formulation has been prepared, the material is subjected to freeze-drying. This can be achieved using a number of commercially available freeze-dryers. These work by freezing the material and then reducing the surrounding pressure to allow the frozen water in the material to sublimate directly from the solid phase to the gas phase. A secondary drying phase may also be carried out. The specification indicate that the lyophilized formulation comprises a moisture content of less than 5%, preferably less than 4%, more preferably less than 3%, even more preferably less than 2%.

The lyophilized formulation may be reconstituted with a diluent such that the antibody concentration in the reconstituted formulation is present in an amount of between 1 and 100 mg/ml, more preferably 1 and 50 mg/ml.

Diluents for use in reconstitution of the lyophilized formulation include sterile water, bacteriostatic water for injection (BWFI), water for injection (WFI) a pH buffered solution such as phosphate buffered saline, sterile saline solution, Ringer's solution or dextrose solution.

A preservative may be added to the diluent to reduce bacterial action in the reconstituted formulation. The specification provides a preservative selected from the group consisting of: octadecyldimethylbenzyl ammonium chloride, hexamethonium chloride, benzalkonium chloride (a mixture of alkylbenzyldimethyl-ammonium chlorides in which the alkyl groups are long- chain compounds), benzethonium chloride, aromatic alcohols such as phenol, butyl and benzyl alcohol, alkyl parabens such as methyl or propyl paraben, catechol, resorcinol, cyclohexanol, 3-pentanol, and m-cresol.

In an aspect of the present disclosure a pharmaceutical formulation is produced as a liquid formulation. Preferably the anti-HER2 antibody or a fragment thereof is present within the liquid formulation in an amount of between 1mg/ml and 100mg/ml, preferably 5mg/ml and 50mg/ml, preferably 10mg/ml and 40mg/ml, more preferably 15mg/ml and 30 mg/ml, even more preferably 20-25mg/ml, especially 21mg/ml.

The liquid formulation can be buffered to a pH of 5.0 - 6.0, preferably pH 5.5 - 5.9, more preferably pH 5.6 - 5.8, even more preferably pH 5.7 ± 0.2, most preferably pH 5.7 ± 0.1, especially pH 5.7. As detailed in Example 1, the applicants have found that an acetate buffer confers significant stability to the liquid formulation, compared to, for example, a citrate buffer. Therefore, the preferred buffer is acetate buffer, more preferably sodium acetate. Preferably, the acetate buffer is present within the liquid formulation in an amount of between 1-50mM, preferably 1-20mM, more preferably 1-10mM, even more preferably 2-7mM, especially 4mM.

Preferably, a tonicifier is present in the liquid formulation at a concentration of 50 to 400mM, preferably 100 to 300mM, more preferably 200 to 250mM, even more preferably 210-220mM, especially 214mM.

Preferably, a surfactant is added to the liquid formulation, which may be selected from polyoxyethylene sorbitan fatty acid esters. Particularly preferably the surfactant is Polysorbate 80 (also known by the brand name Tween 80™ (ICI Americas, Inc.)).

Preferably, the surfactant is present within the liquid formulation in an amount of between 0.001 and 0.1% (w/w), more preferably between 0.002 and 0.05% (w/w), even more preferably between 0.005 and 0.02% (w/w) and especially 0.01% (w/w).

Preferably a solubilizer is added to the liquid formulation. The solubilizer may be proline or glycine, preferably proline. Preferably, the solubilizer is added to the formulation at a concentration of 1-50mg/ml, preferably 5-40mg/ml, more preferably 10-30mg/ml, even more preferably 15-25mg/ml, most preferably at 20mg/ml.

In a preferred aspect the liquid formulation comprises an anti-HER2 antibody or a fragment thereof and pharmaceutically acceptable excipients, buffered to a pH of 5.7 ± 0.2 with acetate buffer. In a further preferred embodiment of the invention, the liquid formulation comprises an anti-HER2 antibody or a fragment thereof formulated with NaCl, proline, Polysorbate 80 and sodium acetate, at a pH of 5.7. Preferably the anti-HER2 antibody or a fragment thereof comprises a heavy chain and light chain of SEQ ID NO: 10 and SEQ ID NO: 11.

In a yet further aspect the liquid formulation comprises 10-40mg/ml of an anti-HER2 antibody or a fragment thereof, 200-250mM NaCl, 10-30mg/ml proline, 0.005 and 0.02% (w/w) Polysorbate 80 and 1-10mM sodium acetate buffer, wherein the pH of the formulation is pH 5.7 ± 0.2. Preferably, the anti-HER2 antibody or a fragment thereof comprises a heavy chain and light chain of SEQ ID NO: 10 and SEQ ID NO: 11.

In a more preferred aspect the liquid formulation comprises 21mg/ml of an anti-HER2 antibody or a fragment thereof, 214mM NaCl, 20mg/ml proline, 0.01% (w/w) Polysorbate 80 and 4mM sodium acetate buffer, wherein the pH of the formulation is pH5.7. Preferably, the anti-HER2 antibody or a fragment thereof comprises a heavy chain and light chain of SEQ ID NO: 10 and SEQ ID NO: 11.

In a further aspect the pharmaceutical formulation comprises a lyophilized formulation of an antibody.

Preferably the anti-HER2 antibody or a fragment thereof is present within the lyophilized formulation in an amount of between 1mg/ml and 100mg/ml, preferably 5mg/ml and 80mg/ml, preferably 10mg/ml and 60mg/ml, preferably 15mg/ml and 40 mg/ml, more preferably 20-40mg/ml, even more preferably 25-35mg/ml, especially 30mg/ml.

The lyophilized formulation can be buffered to a pH of 5.0 - 6.0, preferably pH 5.5 - 5.9, more preferably pH 5.6 - 5.8, even more preferably pH 5.7 ± 0.2, most preferably pH 5.7 ± 0.1, especially pH 5.7. The preferred buffer is acetate buffer, more preferably sodium acetate. Preferably, the acetate buffer is present within the lyophilized formulation in an amount of between 1-50mM, preferably 1-20mM, more preferably 1-10mM, even more preferably 3-7mM, especially 5-6mM.

In a preferred aspect the lyophilized formulation comprises a stabiliser. Preferably, the stabiliser is a lyoprotectant, which is a non-reducing sugar and/or a sugar alcohol. Preferably the lyoprotectant is sucrose and/or mannitol, most preferably sucrose. Preferably the lyoprotectant is present in the lyophilized formulation at a concentration from about 50mM to about 700mM, preferably from about 100mM to about 600mM, more preferably from about 200mM to about 500mM, even more preferably from about 300mM to about 400mM and most preferably at about 350mM.

In a lyophilized formulation comprising sucrose as the lyoprotectant, preferably sucrose is present in the lyophilized formulation in an amount of between 1-90% (w/w), preferably 2-50% (w/w), preferably 4-25% (w/w), more preferably 7-20% (w/w), even more preferably 10-15% (w/w) and most preferably at an amount of 12% (w/w).

In a lyophilized formulation comprising mannitol as the lyoprotectant, preferably mannitol is present in the lyophilized formulation in an amount of between 0.1-20% (w/w), preferably, 0.5-10%, more preferably 1-6%, even more preferably 2-4%, most preferably 2%.

In a lyophilized formulation comprising sucrose as the lyoprotectant, preferably the molar ratio of anti-HER2 antibody to lyoprotectant may be in the range from 100 to about 3000 moles lyoprotectant to 1 mole antibody, preferably from about 500 to about 2500 moles lyoprotectant to 1 mole antibody, more preferably from about 1000 to about 2250 moles lyoprotectant to 1 mole antibody, even more preferably from about 1500 to 2000 moles. Most preferably the molar ratio of anti-HER2 antibody to lyoprotectant may be about 1750 moles lyoprotectant to 1 mole antibody.

Preferably, the lyophilized formulation comprises a bulking agent. Preferably, the bulking agent is glycine present in the lyophilized formulation at a concentration from about 50 to 200mM, preferably from about 100 to 150mM, more preferably from about 120 to 140mM, even more preferably from about 130mM to 135mM. Preferably, the bulking agent is present in the lyophilized formulation in an amount of between 0.1 and 10% (w/w), preferably between 0.2 and 8% (w/w), more preferably between 0.5 and 5% (w/w), even more preferably between 0.7 and 3% (w/w) and most preferably at 1% (w/w).

Preferably, the lyophilized formulation comprises a surfactant, which is selected from polyoxyethylene sorbitan fatty acid esters. Preferably the surfactant is Polysorbate 80 (also known by the brand name Tween 80™ (ICI Americas, Inc.)). Preferably, the surfactant is present within the lyophilized formulation in an amount of between 0.001 and 0.1% (w/w), more preferably between 0.002 and 0.05% (w/w), even more preferably between 0.005 and 0.02% (w/w) and especially 0.014% (w/w).

In a preferred aspect the lyophilized formulation comprises an anti-HER2 antibody or a fragment thereof, stabilisers and a surfactant, buffered to a pH of 5.7 ± 0.2 with acetate buffer. In a further preferred embodiment of the invention, the lyophilized formulation comprises an anti- HER2 antibody or a fragment thereof, glycine, sucrose, Polysorbate 80 and sodium acetate, at a pH of 5.7 ± 0.2. Preferably the anti-HER2 antibody or a fragment thereof comprises a heavy chain and light chain of SEQ ID NO: 10 and SEQ ID NO: 11.

In a further preferred aspect the lyophilized formulation comprises 20-40mg/ml of an anti-HER2 antibody or a fragment thereof, 10-15% (w/w) sucrose, 0.5-5% (w/w) glycine, 0.005-0.02% (w/w) Polysorbate 80 and 1-10mM sodium acetate, wherein the pH of the formulation is pH 5.7 ± 0.2. Preferably the anti-HER2 antibody or a fragment thereof comprises a heavy chain and light chain of SEQ ID NO: 10 and SEQ ID NO: 11.

In a further more preferred aspect the lyophilized formulation comprises 30mg/ml of an anti-HER2 antibody or a fragment thereof, 12% (w/w) sucrose, 1% (w/w) glycine, 0.014% (w/w) Polysorbate 80 and 5.75mM sodium acetate, wherein the pH of the formulation is pH 5.7. Preferably the anti-HER2 antibody or a fragment thereof comprises a heavy chain and light chain of SEQ ID NO: 10 and SEQ ID NO: 11.

In a yet further preferred aspect the lyophilized formulation comprises 20-40mg/ml of an anti-HER2 antibody or a fragment thereof, 300-400mM sucrose, 120-140mM glycine, 0.005-0.02% (w/w) Polysorbate 80 and 1-10mM sodium acetate, wherein the pH of the formulation is pH 5.7 ± 0.2. Preferably the anti-HER2 antibody or a fragment thereof comprises a heavy chain and light chain of SEQ ID NO: 10 and SEQ ID NO: 11.

In a yet further more preferred aspect the lyophilized formulation comprises 30mg/ml of an anti-HER2 antibody or a fragment thereof, 350mM sucrose, 133mM glycine, 0.014% (w/w) Polysorbate 80 and 5.75mM sodium acetate, wherein the pH of the formulation is pH 5.7. Preferably the anti-HER2 antibody or a fragment thereof comprises a heavy chain and light chain of SEQ ID NO: 10 and SEQ ID NO: 11.

Once the lyophilized formulation has been prepared, the material is subjected to freeze-drying. Preferably the formulation following lyophilization comprises a moisture content of less than 5%, preferably less than 4%, more preferably less than 3%, even more preferably less than 2% and especially between 1% and 2%.

In a further aspect the pharmaceutical formulation comprises a reconstituted formulation of an antibody.

The lyophilized formulation may be reconstituted with a diluent such that the concentration of the anti-HER2 antibody or a fragment thereof in the reconstituted formulation is present in an amount of between 1mg/ml and 100mg/ml, preferably 5mg/ml and 50mg/ml, preferably 10mg/ml and 40mg/ml, more preferably 15mg/ml and 30 mg/ml, even more preferably 20 and 25mg/ml, especially 21mg/ml.

Diluents for use in reconstitution of the lyophilized formulation include sterile water, bacteriostatic water for injection (BWFI), water for injection (WFI), a pH buffered solution such as phosphate buffered saline, sterile saline solution, Ringer's solution or dextrose solution. Preferably, the diluent for use in reconstitution of the lyophilized formulation of the present invention is WFI.

The diluent may be added in an amount of between 0.1-100ml, preferably between 1-50ml, more preferably between 2-30ml, even more preferably between 3-15ml, especially between 5-10 ml, to yield an antibody solution at the desired concentration. Preferably, the lyophilized formulation is reconstituted to yield a protein concentration of 21mg/ml with 7.2ml of WFI. Preferably, the reconstituted formulation is buffered to a pH of 5.0 - 6.0, more preferably pH 5.7 ± 0.2, even more preferably 5.7 ± 0.1, most preferably pH 5.7. Preferably, the buffer is acetate buffer, more preferably sodium acetate. Preferably, sodium acetate is present within the reconstituted formulation in an amount of between 1-50mM, preferably 1-20mM, more preferably 1-10mM, even more preferably 2-6mM, especially 4mM.

Preferably, the reconstituted formulation comprises a surfactant, which is selected from polyoxyethylene sorbitan fatty acid esters. Preferably the surfactant is Polysorbate 80 (also known by the brand name Tween 80™ (ICI Americas, Inc.)). Preferably, the surfactant is present within the reconstituted formulation in an amount of between 0.001 and 0.1% (w/w), more preferably between 0.002 and 0.05% (w/w), even more preferably between 0.005 and 0.02% (w/w) and especially 0.01% (w/w).

In a preferred aspect the reconstituted formulation comprises a stabiliser. Preferably, the stabiliser is a lyoprotectant, which is a non-reducing sugar and/or a sugar alcohol. Preferably the lyoprotectant is sucrose and/or mannitol, most preferably sucrose. Preferably the lyoprotectant is present in the reconstituted formulation at a concentration from about 10mM to about 700mM, preferably from about 50mM to about 600mM, more preferably from about 100mM to about 500mM, even more preferably from about 200mM to about 300mM and most preferably at about 225mM to about 275mM, especially about 250mM.

Preferably, the lyoprotectant is sucrose, present in the reconstituted formulation in an amount of between 1-50% (w/w), preferably 2-25% (w/w), preferably 4-20% (w/w), more preferably 5-15% (w/w), even more preferably 6-10% (w/w) and most preferably at an amount of 8.4% (w/w).

In a reconstituted formulation comprising mannitol as the lyoprotectant, preferably mannitol is present in the lyophilized formulation in an amount of between 0.1-20% (w/w), preferably, 0.5-10%, more preferably 1-6%, even more preferably 2-4%, most preferably 2%.

In the reconstituted formulation comprising sucrose as the lyoprotectant, preferably the molar ratio of anti-HER2 antibody to lyoprotectant may be in the range from 100 to about 3000 moles lyoprotectant to 1 mole antibody, preferably from about 500 to about 2500 moles lyoprotectant to 1 mole antibody, more preferably from about 1000 to about 2000 moles lyoprotectant to 1 mole antibody. Most preferably the molar ratio of anti-HER2 antibody to lyoprotectant may be about 1750 moles lyoprotectant to 1 mole antibody.

Preferably, the reconstituted formulation comprises a bulking agent. Preferably, the bulking agent is glycine present in the reconstituted formulation at a concentration from about 50 to 200mM, preferably from about 60 to 150mM, more preferably from about 70 to 120mM, even more preferably from about 80mM to 100mM, most preferably at about 90mM. Preferably, the bulking agent is present in the lyophilized formulation in an amount of between 0.1 and 10% (w/w), preferably between 0.2 and 5% (w/w), more preferably between 0.5 and 2% (w/w), even more preferably at 0.7% (w/w).

In a preferred aspect the reconstituted lyophilized formulation comprises an anti- HER2 antibody or a fragment thereof, stabilisers, a surfactant and a diluent, buffered to a pH of 5.7 ± 0.2 with acetate buffer. In a further preferred embodiment of the invention, the reconstituted lyophilized formulation comprises an anti- HER2 antibody or a fragment thereof, glycine, sucrose, Polysorbate 80, WFI and sodium acetate at a pH of 5.7 ± 0.2. Preferably the anti-HER2 antibody or a fragment thereof comprises a heavy chain and light chain of SEQ ID NO: 10 and SEQ ID NO: 11.

In a preferred aspect the reconstituted lyophilized formulation comprises 15-30mg/ml of an anti-HER2 antibody or a fragment thereof, 5-15% (w/w) sucrose, 0.5-2% (w/w) glycine, 0.005-0.02% (w/w) Polysorbate 80, WFI and 1-10mM sodium acetate, wherein the pH of the formulation is pH 5.7 ± 0.2. Preferably, the anti-HER2 antibody or a fragment thereof comprises a heavy chain and light chain of SEQ ID NO: 10 and SEQ ID NO: 11.

In a more preferred aspect the reconstituted lyophilized formulation comprises 21mg/ml of an anti-HER2 antibody or a fragment thereof, 8.4% (w/w) sucrose, 0.7% (w/w) glycine, 0.01% (w/w) Polysorbate 80, WFI and 4mM sodium acetate, wherein the pH of the formulation is pH 5.7. Preferably, the anti-HER2 antibody or a fragment thereof comprises a heavy chain and light chain of SEQ ID NO: 10 and SEQ ID NO: 11.

In a further preferred aspect the reconstituted lyophilized formulation comprises 15-30mg/ml of an anti-HER2 antibody or a fragment thereof, 225-275mM sucrose, 80-100mM glycine, 0.005-0.02% (w/w) Polysorbate 80, 5-10ml WFI and 1-10mM sodium acetate buffer, wherein the pH of the formulation is pH 5.7 ± 0.2. Preferably, the anti-HER2 antibody or a fragment thereof comprises a heavy chain and light chain of SEQ ID NO: 10 and SEQ ID NO: 11.

In a further more preferred aspect the reconstituted lyophilized formulation comprises 21mg/ml of an anti-HER2 antibody or a fragment thereof, 246mM sucrose, 90mM glycine, 0.01% (w/w) Polysorbate 80, 7.2ml WFI and 4mM sodium acetate buffer, wherein the pH of the formulation is pH 5.7. Preferably, the anti-HER2 antibody or a fragment thereof comprises a heavy chain and light chain of SEQ ID NO: 10 and SEQ ID NO: 11.

According to a further aspect there is provided a use of a pharmaceutical antibody formulation as defined herein for the treatment of a HER2 related disorder. Preferably, the HER2 related disorder is selected from cancer. The specification provides examples of cancer to be treated herein selected from the group consisting of, but are not limited to, carcinoma, lymphoma, blastoma, sarcoma, and leukemia or lymphoid malignancies. Particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. More particular examples include a HER2 positive cancer such as metastatic breast cancer, early breast cancer and metastatic gastric cancer. Most preferably, the HER2 related disorder is HER2 positive metastatic breast cancer.

The disclosure further provides a pharmaceutical antibody formulation as defined herein, for use as treatment or prophylaxis of a HER2 related disorder.

The disclosure further provides a pharmaceutical antibody formulation as defined herein for use in the treatment of a HER2 related disorder.

The pharmaceutical formulation may be a liquid formulation, a lyophilized formulation or a reconstituted formulation i.e. a lyophilized formulation which is reconstituted before use. A liquid formulation is usually provided in the form of containers with defined volume, including sealed and sterilized plastic or glass vials, ampoules and syringes, as well as in the form of large volume containers like bottles. A lyophilized formulation is usually provided in the form of a powder of a defined weight in sealed and sterilized plastic or glass vials. The powder is reconstituted before use. Preferably, the pharmaceutical formulation of the invention is a lyophilized formulation. More preferably the pharmaceutical formulation of the invention is a reconstituted formulation. The pharmaceutical formulation of the invention may be administered orally, or by injection (for example, subcutaneously, intravenously, intraperitoneal or intramuscularly), or by inhalation or topically (for example intraocular, intranasal, rectal, into wounds, on skin). The route of administration can be determined by the physicochemical characteristics of the treatment, by special considerations for the disease or by the requirement to optimise efficacy or to minimise side-effects. Preferably, the formulation of the invention is administered by intravenous infusion, e.g., as a bolus and/or by continuous infusion over a period of time.

The specification discloses also an article of manufacture comprising:
(a) a container which holds a lyophilized formulation of an anti-HER2 antibody or a fragment thereof, a lyoprotectant and an acetate buffer; and
(b) instructions for reconstituting the lyophilized formulation with a diluent to an antibody concentration in the reconstituted formulation of about 10-40mg/ml.

Preferably, the lyophilized formulation is reconstituted with a diluent to yield an antibody concentration of about 12-30mg/ml. More preferably, the antibody concentration is about 15-25mg/ml, even more preferably about 21mg/ml.

Formulations provided may be administered to individuals. Administration is preferably in a "therapeutically effective amount", this being sufficient to show benefit to a subject. Such benefit may be at least amelioration of at least one symptom. The actual amount administered, and rate and time-course of administration, will depend on the nature and severity of what is being treated. Prescription of treatment, e.g. decisions on dosage etc, is within the responsibility of medical doctors. Appropriate doses of antibody are well known in the art (Ledermann JA et al., (1999) Int J Cancer 47: 659-664; Bagshawe KD et al., (1991) Antibody, Immunoconjugates and Radiopharmaceuticals, 4: 915-922). The specification provides indications on dosage for administration, even if the precise dose will depend upon a number of factors, including the size and location of the area to be treated, body weight of the subject, the precise nature of the antibody (e.g. whole antibody or fragment) and any additional therapeutic agents administered before, at the time of or after administration of the antibody. A typical antibody dose will be in the range 2mg/kg to 8mg/kg for intravenous administration.

The antibody or a fragment thereof is suitably administered to the subject at one time or over a series of treatments. Depending on the type and severity of the disease, about 0.1mg/kg to 15mg/kg of antibody is an initial candidate dosage for administration to the subject, for example, by one or more separate administrations, or by continuous infusion. A typical daily dosage might range from about 0.1mg/kg to 50mg/kg or more, depending on the factors mentioned above. For repeated administrations over several days or longer, depending on the condition, the treatment is sustained until a desired suppression of disease symptoms occurs. The preferred dosage of the antibody will be in the range from about 0.05mg/kg to about 10mg/kg. Thus, one or more doses of about 0.5mg/kg, 2.0mg/kg, 4.0mg/kg or 10mg/kg (or any combination thereof) may be administered to the subject. Such doses may be administered intermittently, e.g. every week or every three weeks (e.g. such that the subject receives from about two to about 20, e.g. about six doses of the anti-HER2 antibody). An initial higher loading dose, followed by one or more lower doses may be administered. An exemplary dosing regimen comprises administering an initial loading dose of about 4mg/kg to 8mg/kg, followed by a weekly maintenance dose of about 2mg/kg to 6mg/kg of the anti-HER2 antibody. Preferably the initial loading dose is 8mg/kg followed by a weekly or three weekly dose of 2mg/kg or 6mg/kg, respectively.

Other therapeutic regimens may be combined with the administration of the anti-HER2 antibody or a fragment thereof. The combined administration includes co-administration, using separate formulations or a single pharmaceutical formulation, and consecutive administration in either order, wherein preferably there is a time period while both (or all) active agents simultaneously exert their biological activities.

It may also be desirable to combine administration of the anti-HER2 antibody or a fragment thereof, with administration of an antibody or a fragment thereof directed against another tumor associated antigen. The other antibody in this case may, for example, bind to EGFR, ErbB3, ErbB4, or vascular endothelial growth factor (VEGF).

In one aspect the treatment of the present invention involves the combined administration of an anti-HER2 antibody or a fragment thereof and one or more chemotherapeutic agents or growth inhibitory agents, including co-administration of cocktails of different chemotherapeutic agents. Preferred chemotherapeutic agents include taxanes (such as paclitaxel and docetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M. C. Perry, Williams & Wilkins, Baltimore, Md. (1992). The antibody may be combined with an anti-hormonal compound, e.g., an anti-oestrogen compound such as tamoxifen, an anti-progesterone such as onapristone (see EP616812), or an anti-androgen such as fiutamide, in dosages known for such molecules. Where the cancer to be treated is hormone independent cancer, the subject may previously have been subjected to anti-hormonal therapy and, after the cancer becomes hormone independent, the anti-HER2 antibody or a fragment thereof (and optionally other agents as described herein) may be administered to the subject.

In some cases it may be beneficial to also co-administer a cardioprotectant (to prevent or reduce myocardial dysfunction associated with the therapy) or one or more cytokines to the subject. One may also co-administer an EGFR-targeted drug or an anti-angiogenic agent. In addition to the above therapeutic regimes, the subject may be subjected to surgical removal of cancer cells and/or radiation therapy.

### Examples

### Example 1: Development of a liquid formulation

The purpose of this work was to determine the main formulation conditions that have an impact on the stability of the anti-HER2 antibody in a liquid formulation, which can be used as the basis for a lyophilized formulation of the antibody.

### General Materials & Methods

The anti-HER2 antibody was produced in wave bioreactors and captured by Protein A using MabSelect Sure (GE Healthcare; www.gelifesciences.com). The product was polished by preparative size exclusion chromatography using the column HiLoad 16/60 Sephadex 200pg, 120ml (GE Healthcare) with the running buffer 10mM citrate pH 5.5. The product was concentrated to 50mg/ml by UFDF before the addition of excipients. CEX-HPLC was performed with a column Bakerbond Wide-Pore CBX 5µm 4.6x250mm 300A (JT Baker 7114-00; www.jtbaker.nl) followed by SEC-HPLC using the column BioSep S3000 300mm x 4.6mm, 300A, separation range: 5-500 kDa (Phenomenex 00H-2146-E0; www.phenomenex.com).

The following chemicals were used in the formulation screening: sodium acetate trihydrate (Merck; www.merckmillipore.com), acetic acid (Sigma; www.sigmaaldrich.com), sodium citrate (Sigma), ammonium sulphate (Fluka; www.sigmaaldrich.com), sodium chloride (Merck), hydrochloric acid 37% (Sigma) and sodium hydroxide 50% (Sigma). The following excipients were used in the formulation screening: Tween80™ (VWR; https://uk.vwr.com), sorbitol (Sigma), PEG8000 (Sigma), glycerol (VWR prolabo), sucrose (Sigma), mannitol (Merck) and glycine (Merck), and the amino acids: glutamine (Fluka), proline (Merck), leucine (Merck), aspartic acid (Merck), methionine (Sigma) and histidine (Sigma). All chemicals and excipients used during these screens were pharmacopeia grade (US or EP), except for sodium hydroxide 50% solution, and are suitable as parenterals. Buffer and excipients were filtered through a 0.22µm filter before use.

### 1.1: Screen 1

*Aim:* The conditions tested were: pH, buffer concentration, tonicifier concentration, surfactant concentration, amino acids and polyols.

*Materials& Methods:* The anti-HER2 antibody was prepared as detailed above in the General Materials & Methods section. Formulated samples of the anti-HER2 antibody were incubated at 5±3°C in a cold room, at 22±5°C (laboratory room temperature, uncontrolled temperature) and at 37±1°C in a cell culture incubator (Hera Cell 150, Thermo (R-022) or equivalent). The buffer exchange for the 50mg/ml concentrated anti-HER2 antibody samples was performed with PD-10 columns (GE Healthcare). The formulation conditions were: pH 5.00 to 8.00, buffer concentration from 10 to 50mM, amino acid concentration from 0 to 20g/L, amino acid type (glutamine, proline, leucine, aspartic acid), sucrose 0 to 10g/L, (NH₄)₂SO₄ concentration from 0 to 15g/L and Tween 80™ concentration from 0 to 0.01%. The buffers tested were: acetate pH range 3.7-5.7; histidine pH range 5.0-7.0; citrate pH range 5.4-7.4 and phosphate pH range 6.2-8.2. The final volume of the anti-HER2 antibody was adjusted to reach the 21mg/ml target concentration.

*Procedure:* The anti-HER2 antibody was formulated at the target concentration of 21g/L in 32 formulation formulations designed on the statistical software JMP® (SAS). The samples were incubated at 37±1°C for 1 month and were analysed by SEC-HPLC and CEX-HPLC. A design of experiment approach was used with the statistical software JMP®. Table 1 overleaf shows the buffer, pH range and buffer concentrations tested.

*Results:* Fragmentation and aggregation were visible in most samples; however the only parameter that had a major impact on the product stability was pH. In the pH range of pH 5.00 to 6.00, more preferably about pH 5.50, the maximal proportion of non-deamidated anti-HER2 antibody was obtained. This pH range (pH 5.00 to 6.00) was used in the further experiments detailed herein.

**Table 1: JMP design for the first screening step. The design produced 32 conditions with various concentrations of buffer in a pH range of 5.00 to 8.00**

| **No** | **pH** | **buffer conc (mM)** | **buffer** |
|---|---|---|---|
| 1 | 5.45 | 18.0 | acetate |
| 2 | 7.40 | 10.0 | phosphate |
| 3 | 5.00 | 10.0 | acetate |
| 4 | 8.00 | 30.0 | phosphate |
| 5 | 7.55 | 50.0 | phosphate |
| 6 | 8.00 | 10.0 | phosphate |
| 7 | 8.00 | 44.0 | phosphate |
| 8 | 7.70 | 40.0 | phosphate |
| 9 | 6.50 | 50.0 | histidine |
| 10 | 5.60 | 50.0 | histidine |
| 11 | 5.00 | 10.0 | acetate |
| 12 | 7.55 | 34.0 | phosphate |
| 13 | 5.45 | 14.0 | acetate |
| 14 | 8.00 | 18.0 | phosphate |
| 15 | 5.90 | 10.0 | histidine |
| 16 | 6.20 | 26.0 | histidine |
| 17 | 5.45 | 32.0 | acetate |
| 18 | 5.00 | 50.0 | acetate |
| 19 | 7.40 | 16.0 | phosphate |
| 20 | 6.80 | 26.0 | histidine |
| 21 | 5.00 | 16.0 | acetate |
| 22 | 7.40 | 50.0 | phosphate |
| 23 | 5.00 | 28.0 | acetate |
| 24 | 6.50 | 16.0 | phosphate |
| 25 | 7.25 | 34.0 | phosphate |
| 26 | 8.00 | 16.0 | phosphate |
| 27 | 5.00 | 44.0 | acetate |
| 28 | 5.45 | 44.0 | acetate |
| 29 | 6.35 | 44.0 | histidine |
| 30 | 5.60 | 42.0 | histidine |
| 31 | 8.00 | 50.0 | phosphate |
| 32 | 6.65 | 42.0 | histidine |

### 1.2: Screen 2

*Aim:* Various excipients were tested for their efficiency in stabilizing the product in the optimal pH range determined in Screen 1.

*Materials* & *Methods:* The anti-HER2 antibody was prepared as detailed above in the General Materials & Methods section. Formulated samples of the anti-HER2 antibody were incubated at 37±1°C in a cell culture incubator, Hera Cell 150, Thermo (R-022) or equivalent. Buffer exchange for the 50mg/ml concentrated anti-HER2 antibody samples was performed with PD-10 columns (GE Healthcare). The final volume was adjusted to reach the 21 mg/ml target concentration. The parameters evaluated were: various concentrations of asparagine, sucrose, PEG8000, ammonium sulphate, sodium chloride, glutamine, ethanol and Tween 80™.

*Procedure:* The anti-HER2 antibody was formulated at the target concentration of 21g/L or 50g/L. The tested formulations are shown in Table 2 overleaf. Various excipients were tested for their efficiency in stabilizing the product. The samples were stressed by an accelerated study at 37°C for one month and analyzed by SEC-HPLC and CEX-HPLC.

*Results:* Figure 1a shows that the variations of deamidation rate formulations were small. At time 0, the product had 75% of the non-deamidated form and after one month at 37°C, all the samples had from 36% to 43% of non-deamidated form. Formulation of the anti-HER2 antibody at a high concentration in the presence of a high concentration of Tween80™ or in a high salt concentration produced the best deamidation results. Figures 1b, c and d show that the conditions producing the lowest deamidation produced the highest aggregation levels and also the highest loss of antibody solubility. In conclusion, it was possible to reduce the deamidation rate to a low extent by using extreme formulation compositions. These included high concentrations of protein, salt and surfactant. The best conditions produced an increase of 6% of the non-deamidated form; however the same conditions produced the largest proportion of aggregation and an important loss in solubility.

**Table 2: All samples were in 10mM citrate pH 5.5 and 50mM NaCl to which were added various excipients. The antibody was formulation at 21g/L or 50g/L.**

| Sample No | Base composition | Formulation composition | Mab concentration (g/L) |
|---|---|---|---|
| 1 | | Sucrose 1g/L | 21 |
| 2 | | Sucrose 5g/L | 21 |
| 3 | | Sucrose 10g/L | 21 |
| 4 | | Sucrose 25g/L | 21 |
| 5 | | Glycerol 1% | 21 |
| 6 | | Glycerol 5% | 21 |
| 7 | | Glycerol 10% | 21 |
| 8 | | Glycerol 25% | 21 |
| 9 | | PEG8000 3% | 21 |
| 10 | | PEG8000 1.5% | 21 |
| 11 | | NaCl 50mM | 50 |
| 12 | | NaCl 100mM | 50 |
| 13 | 10mM citrate pH 5.5 50mM NaCl (all compositions) | NaCl 100mM | 21 |
| 14 | | NaCl 250mM | 21 |
| 15 | | NaCl 500mM | 21 |
| 16 | | (NH₄)₂SO₄ 100mM | 21 |
| 17 | | Ethanol 1% | 21 |
| 18 | | Ethanol 5% | 21 |
| 19 | | Ethanol 10% | 21 |
| 20 | | Tween80 0.01% | 21 |
| 21 | | Tween80 0.1% | 21 |
| 22 | | Tween80 1.0% | 21 |
| 23 | | Tween80 2.5% | 21 |
| 24 | | Asparagine 1g/L | 21 |
| 25 | | Asparagine 5g/L | 21 |
| 26 | | Asparagine 10g/L | 21 |
| 27 | | Glutamine 1g/L | 21 |
| 28 | | Glutamine 5g/L | 21 |
| 29 | | Glutamine 10g/L | 21 |

### 1.3: Screen 3

*Aim:* The aim of this screen was to reduce the deamidation of an anti-HER2 antibody by fine tuning the best parameters from Screen 2.

*Materials* & *Method:* The anti-HER2 antibody was prepared as detailed above in the General Materials & Methods section. For accelerated studies, the formulated anti-HER2 antibody samples were incubated at 37±1°C in a cell culture incubator, Hera Cell 150, Thermo (R-022) or equivalent. The parameters tested were: pH 4.00 to pH 6.00, buffer types were acetate or citrate and the salt concentration was either 100mM or 250mM. The conditions are detailed in Table 3 overleaf. From a starting sample concentration of 84mg/ml concentrated solutions of NaCl and buffer were added to reach the desired concentrations. The final volume was adjusted to reach the 21mg/ml, 42mg/ml or 63mg/ml target concentrations of anti-HER2 antibody.

*Procedure:* The anti-HER2 antibody was formulated at the target concentrations of 21mg/ml, 42mg/ml or 63mg/ml. Two buffer types with acidic pKas were evaluated for their efficiency in stabilizing the product in the optimal pH range of pH 4.00 to pH 6.00. The samples were stressed for one month in an accelerated study at 37°C and analyzed by SEC-HPLC and CEX-HPLC.

*Results:* From the previous screens it was observed that the best anti-HER2 antibody stability results were obtained at mildly acidic pH values. The deamidation could be mostly inhibited in the presence of higher antibody concentration or salt concentration. In this screen, formulations of the anti-HER2 antibody at high antibody concentration or salt concentration and at a mildly acidic pH were evaluated. At time 0, the product had 75% of non-deamidated form. In Figure 2a, the CEX-HPLC results shown that the maximal percentage of non-deamidated form reduced to 45%. The deamidation was more important at the very low pH values and an optimal pH was found to be in the range of pH 5.0 to pH6.0. The extent of deamidation at low pH was reduced when the buffer was acetate as opposed to citrate. NaCl at a high concentration (250mM) reduced deamidation at low pH values. Altering the concentration of the anti-HER2 antibody in the formulations did not impact the results.

The SEC-HPLC results (Figures 2b, c, d & e) showed a maximum degradation at low pH values and high salt concentrations. At pH 4.0 and pH 4.5 there were minimal amounts of antibody monomeric form, high aggregation and high fragmentation. These low pH degradations were more pronounced when the buffer was acetate. The best results were obtained at pH 5.5 and pH 6.0, where acetate and citrate were found to be equivalent in stabilizing the anti-HER2 antibody. NaCl at 250mM produced more degradation at low pH values than NaCl at 100mM. The concentration of anti-HER2 antibody did not produce any difference.

**Table 3: Testing conditions for Screen 3 to evaluate two buffers: citrate and acetate in a pH range of pH 4.0 to pH 6.0**

| **No** | **antibody conc. (mg/ml)** | **Buffer** | **pH** | **NaCl (mM)** |
|---|---|---|---|---|
| 1 | 21 | 10mM citrate | 4.0 | 100 |
| 2 | 21 | | 4.5 | 100 |
| 3 | 21 | | 5.0 | 100 |
| 4 | 21 | | 5.5 | 100 |
| 5 | 21 | | 6.0 | 100 |
| 6 | 42 | 10mM citrate | 4.0 | 100 |
| 7 | 42 | | 4.5 | 100 |
| 8 | 42 | | 5.0 | 100 |
| 9 | 42 | | 5.5 | 100 |
| 10 | 42 | | 6.0 | 100 |
| 11 | 63 | 10mM citrate | 4.0 | 100 |
| 12 | 63 | | 4.5 | 100 |
| 13 | 63 | | 5.0 | 100 |
| 14 | 63 | | 5.5 | 100 |
| 15 | 63 | | 6.0 | 100 |
| 16 | 21 | 10mM acetate | 4.0 | 100 |
| 17 | 21 | | 4.5 | 100 |
| 18 | 21 | | 5.0 | 100 |
| 19 | 21 | | 5.5 | 100 |
| 20 | 21 | | 6.0 | 100 |
| 21 | 42 | 10mM acetate | 4.0 | 100 |
| 22 | 42 | | 4.5 | 100 |
| 23 | 42 | | 5.0 | 100 |
| 24 | 42 | | 5.5 | 100 |
| 25 | 42 | | 6.0 | 100 |
| 26 | 63 | 10mM acetate | 4.0 | 100 |
| 27 | 63 | | 4.5 | 100 |
| 28 | 63 | | 5.0 | 100 |
| 29 | 63 | | 5.5 | 100 |
| 30 | 63 | | 6.0 | 100 |
| 31 | 21 | 10mM citrate | 4.0 | 250 |
| 32 | 21 | | 4.5 | 250 |
| 33 | 21 | | 5.0 | 250 |
| 34 | 21 | | 5.5 | 250 |
| 35 | 21 | | 6.0 | 250 |
| 36 | 42 | 10mM citrate | 4.0 | 250 |
| 37 | 42 | | 4.5 | 250 |
| 38 | 42 | | 5.0 | 250 |
| 39 | 42 | | 5.5 | 250 |
| 40 | 42 | | 6.0 | 250 |

In conclusion, the most important parameter impacting the formulation was the pH of the sample. The best stability pH range was found in the range of pH 5.5 and pH 6.0. The antibody concentration did not produce a significant difference on deamidation.

### 1.4: Screen 4

*Aim:* The aim of this step was to define a final liquid formulation that could be used as the basis for the lyophilized formulation. The first step consisted of narrowing down the best parameters to their optimal range. The second step was the design of an experiment using statistical software for selecting a single final composition for the anti-HER2 antibody liquid formulation.

*Materials & Methods:* Anti-HER2 antibody was prepared as detailed above in the General Materials & Methods section. For accelerated studies, the formulated samples of the anti-HER2 antibody were incubated at 5±3°C in a cold room, at 22±5°C (laboratory room temperature, uncontrolled temperature) and 37±1°C in a cell culture incubator, Hera Cell 150, Thermo (R-022) or equivalent. The statistical software used was JMP® (SAS).

*Procedure:* Preliminary screenings were performed in order to narrow down the parameters for the experimental design. The buffer screening was performed by dialysing the anti-HER2 antibody in NaCl 50mM with the addition of acetate or citrate at pH 5.5 at the following concentrations: 1.0, 2.5, 5.0, 7.5, 10.0, 15.0 and 20.0mM. The optimal salt concentration was determined by dialysing the anti-HER2 antibody in 10mM citrate pH 5.5 with the addition of the following concentrations of NaCl: 0, 10, 50, 100, 200, 400, 600, 800 and 1000mM. The antioxidant methionine was tested at the concentrations of 1, 4 or 8mM in the anti-HER2 antibody dialysed in 5mM Citrate pH 5.5, 50mM NaCl. The experiment was performed according to a statistical design. The parameter ranges used in the statistical software JMP® were: pH 5.50 to pH 6.25, buffer concentration from 0 to 20mM, NaCl concentration from 50mM to 250mM, a polyol (either sorbitol, glycerol or none), an amino acid (proline, glycine or none), Tween80™ 0% or 0.01%, methionine at 0mM or 4mM. The design of the experimental approach is shown in Table 4 below.

**Table 4: JMP® statistical software design for the experiment. The design produced 24 conditions.**

| **No** | **NaCl (mM)** | **Acetate (mM)** | **pH** | **Sugar (5%)** | **Amino acid (20g/L)** | **Tween80® %** | **Methionine (mM)** |
|---|---|---|---|---|---|---|---|
| 1 | 50 | 15.3 | 6.0 | Sorbitol | none | 0.010 | 0 |
| 2 | 230 | 7.7 | 6.1 | Sorbitol | Pro | 0.010 | 4 |
| 3 | 50 | 1.0 | 6.2 | Glycerol | none | 0.000 | 0 |
| 4 | 180 | 7.7 | 5.9 | Glycerol | Gly | 0.000 | 0 |
| 5 | 120 | 7.7 | 5.7 | Glycerol | Gly | 0.010 | 4 |
| 6 | 250 | 11.5 | 5.6 | none | Gly | 0.010 | 4 |
| 7 | 120 | 13.4 | 5.6 | Sorbitol | none | 0.000 | 4 |
| 8 | 180 | 1.0 | 5.9 | Sorbitol | Pro | 0.000 | 4 |
| 9 | 150 | 2.9 | 5.5 | none | none | 0.000 | 0 |
| 10 | 250 | 20.0 | 6.2 | Sorbitol | Gly | 0.000 | 4 |
| 11 | 220 | 20.0 | 6.0 | none | Pro | 0.010 | 0 |
| 12 | 50 | 5.8 | 5.4 | Glycerol | Pro | 0.010 | 4 |
| 13 | 80 | 20.0 | 5.7 | none | none | 0.010 | 4 |
| 14 | 250 | 2.9 | 5.7 | none | none | 0.000 | 0 |
| 15 | 230 | 15.3 | 5.6 | Glycerol | Gly | 0.000 | 0 |
| 16 | 190 | 13.4 | 5.6 | Sorbitol | Pro | 0.010 | 0 |
| 17 | 70 | 12.4 | 5.5 | Sorbitol | Gly | 0.000 | 4 |
| 18 | 70 | 18.1 | 6.2 | none | Pro | 0.000 | 0 |
| 19 | 140 | 18.1 | 5.9 | none | Gly | 0.010 | 4 |
| 20 | 250 | 18.1 | 6.2 | Sorbitol | Gly | 0.010 | 0 |
| 21 | 50 | 20.0 | 6.0 | Glycerol | Pro | 0.000 | 4 |
| 22 | 240 | 1.0 | 6.2 | Glycerol | none | 0.010 | 4 |
| 23 | 80 | 3.9 | 6.1 | none | Gly | 0.000 | 4 |
| 24 | 70 | 17.2 | 5.5 | Sorbitol | Gly | 0.010 | 0 |

*Results:* The results are shown in Figure 3 and Table 5. Figure 3a shows that the aggregation and fragmentation increased at NaCl concentrations higher than 200mM. Figure 3b shows that the proportion of non deamidated forms was lowest at 0mM to 50mM NaCl and then increased linearly with the salt concentration. Figure 3c showed that at pH 5.5, an acetate buffer resulted in less fragments and aggregates. The antioxidant methionine had no impact on the stability of the anti-HER2 antibody.

**Table 5: Anti-HER2 antibody formulated with an antioxidant: percentage of monomers, aggregation, fragmentation and non deamidated form**

| **Methionine (mM)** | **Conc (mg/ml)** | **% monomer** | **% dimer** | **% fragment** | **% non-deamidated** |
|---|---|---|---|---|---|
| 0.0 | 17.828 | 98.56 | 0.86 | 0.37 | 42.16 |
| 1.0 | 18.230 | 98.83 | 0.61 | 0.36 | 42.03 |
| 4.0 | 18.473 | 98.74 | 0.87 | 0.39 | 41.50 |
| 8.0 | 18.374 | 98.56 | 0.81 | 0.39 | 41.24 |

Therefore, for the final experimental design, it was decided to include NaCl in a range of 50mM to 250mM and an acetate buffer in a range of 1.0 mM to 20.0mM. A single concentration of methionine was also included at a concentration of 4.0 mM.

The final liquid formulation composition of the anti-HER2 antibody was determined in an experiment using the statistical software JMP® as shown in Table 4. The samples were incubated at 37°C for 1 month and were analyzed by SEC-HPLC and CEX-HPLC. The results were entered in the statistical software, which detected statistical significance (P < 0.05) for the percentage of monomers, percentage of aggregates and product concentration (results not shown). The responses were of excellent quality with R squares of 1.00, small probability of wrong rejection of the null hypothesis (P < 0.05) and small RMSE values. The fragments response had a R square of 0.94 but the RMSE and P values were large. The percentage of fragments had no statistically significant relation to any buffer composition.

The prevision of profiles (results not shown) indicated that the polyol, amino acid, methionine and Tween80™ had very large error bars and no conclusions could be made. pH, buffer concentration and salt concentration had the largest impact on the product stability. The pH was optimal for minimum aggregation in the range of pH 5.9 to pH 6.1 but the optimum for minimum deamidation was at pH 5.5 to pH 5.8. The optimal NaCl concentration for low aggregation was in the range of 75mM to 175mM and the optimum for minimum deamidation was 200mM to 225mM. The optimal buffer concentrations predicted for minimal aggregation were in the range of 7.5mM to 17.5mM and the optimum for minimal deamidation were with buffer concentrations less than 5mM. In short, the optimum conditions for avoiding deamidation were different to the optimum conditions for avoiding aggregation.

During the screening experiments the aggregation levels were much lower than the deamidation levels. After a one month accelerated study at 37°C at pH 5.5 the aggregation in the screenings discussed above was generally in the range of 0.5% while the deamidated form increased by 30%. It was decided to formulate in conditions that would reduce the largest degradation / deamidation. Therefore, it was decided that the final composition would contain: 4mM acetate pH 5.7 and 214mM NaCl. Since these conditions are less favourable for anti-HER2 antibody solubility, an excipient with solubilising properties was also included and proline at 20g/L was added to the final composition. Finally, although not demonstrated, the presence of a surfactant is known to be desirable in a formulation to avoid product loss due to the mechanical stress encountered during transport (Mahler H-C et al. (2009) J Pharm Sci, 98(12): 4525-33). During the screening experiments Tween80™ at a low concentration improved the solubility and did not impact negatively the integrity of the product. Therefore Tween80™ at 0.01% was added to the final composition.

The parameter that was the most import for stability of the anti-HER2 antibody was pH and therefore an acetate buffer was chosen for the final concentration as this had been shown to confer greater stability on the formulation than a citrate buffer (see Fig 3c). The optimal concentrations of tonicifier and buffer were detected in the optimal pH range. Amino acids were also tested with some having useful solubilising properties on the anti-HER2 antibody. The surfactants tested had no negative impact on the anti-HER2 antibody stability at lower concentrations and were therefore included in the final composition for protection against mechanical stress. The final composition is shown in Table 6.

**Table 6: Anti-HER2 antibody liquid formulation**

| **Liquid formulation** | |
|---|---|
| anti-HER2 Ab | 21g/L |
| pH | pH 5.7 |
| buffer | 4mM acetate |
| tonicifier | 214mM NaCl |
| surfactant | 0.01% Tween80™ |
| solubilizer | 20g/L proline |

### Example 2: Development of a Lyophilized formulation

The liquid formulation of the anti-HER2 antibody as determined in Example 1, was used at the starting point for developing an optimal lyophilized formulation of the same antibody. It was clearly demonstrated in the screens of Example 1, that the most important parameter for the stability of this anti-HER2 antibody in formulation was pH. Use of an acetate buffer in the form of sodium acetate was found to give optimal results compared to a citrate buffer and therefore the acetate buffer was included in the final liquid formulation. To further stabilise the formulation of the anti-HER2 antibody for lyophilisation a lyoprotectant was required. As detailed herein, many types of lyoprotectants are available and for the purposes of this experiment, the following lyoprotectants were initially selected: sucrose, mannitol, glucose, sorbitol and lactose. However, it is widely known that glucose, lactose and sorbitol are reducing sugars and therefore these lyoprotectants were not selected for further experimental work. For the first runs, mannitol was chosen as the lyoprotectant for the anti-HER2 antibody lyophilized formulation.

### General Materials & Methods

The following chemicals were used in the formulation screening: sodium acetate trihydrate (Merck), acetic acid (Sigma), hydrochloric acid 37% (Sigma) and sodium hydroxide 50% (Sigma). The following excipients were used in the formulation screening: Tween80™ (VWR), sucrose (Sigma), mannitol (Merck) and glycine (Merck). Stock solutions of sucrose (600g/l), glycine (130g/l), mannitol (150g/l) and Tween80™ (10%) were prepared in 5.75mM sodium acetate pH 5.7. HCl 37% and NaOH were used for pH adjustment. The excipients were then added to the anti-HER2 antibody formulation by dilution of the stock solution in the formulation to achieve the desired concentration. Specifications for the buffer were 5.7 ± 0.1 for the pH and 0.42 to 0.66mS/cm for the conductivity. ISO3696 Type II water was used throughout. This highly purified water was prepared in-house using an osmotic membrane and an ozone sterilization system (Christ Aqua, Switzerland). All chemicals and excipients were suitable for parenteral administration. The buffer was filtered through a 0.22µm filter before use.

The anti-HER2 antibody test material used for this study was first purified using Protein A affinity chromatography using MabSelect Sure (GE Healthcare). As the purity of the product post protein A was high (monomer content >99%), no further purification steps were performed prior to diafiltration and concentration of the product.

Purified anti-HER2 antibody was diafiltrated 7-10 times by tangential flow filtration (TFF) in 5.75mM sodium acetate pH 5.7 and then concentrated to 50-60g/l. After TFF, the anti-HER2 antibody was formulated by the addition of sucrose, glycine and Tween80™ to reach the final buffer composition of 30g/l. The reference standard material used for the analytical testing was DRS-anti-HER2 antibody and was kept at Quality Control at a temperature below -60°C.

To prepare the lyophilized drug product, a Telstar lyophilization system was used (Lyobeta 15 lyophilizer; Telstar, Terrassa, Spain). A VP600 Vötsch stability chamber (Vötsch Industrietechnik GmbH, Germany) was used for the 3 months study at 40 ± 2°C, 50 ± 5% relative humidity test and also for the 12 months study at 25 ± 2°C, ± 5% relative humidity test. A standard laboratory fridge was used for the 36 months stability at 5 ± 3°C.

Vials and stopper materials used were those intended for clinical trial manufacture. Vials were 20 ml Fiolax clear, USP type I glass vials purchased from Schott (Art. No: 1156521) and the stoppers were Flurotec distributed by West Pharma (ref FD20TT3WRS).

Stability of the drug product was determined using a number of tests to evaluate changes in the drug product over time. CEX-HPLC (Dionax, ProPac WCX-104 x 250mm), SEC-HPLC (Phenomenex, YARRA 3u SEC-3000 7.8 x 300 mm) and SDS-PAGE are sensitive indicators of product breakdown. The profile of drug product using these assays will change over time if the sample is subject to degradation. The A280 assay can be used to detect changes in the protein level which may be indicative of product breakdown or adhesion to the container wall. An ELISA cell based assay and an ELISA binding assay can be used to determine the potency of the product, with a change in the potency of the sample being indicative of product deterioration. The residual moisture of the product was assessed by Karl Fisher assay.

### 2.1: Screen 1

*Aim:* To determine the effect of the lyoprotectant mannitol on antibody stability

*Materials* & *Method:* The formulations were prepared according to the General Materials & Methods section above. Two formulations were tested in this screen that contained mannitol with different concentrations of Tween80™. The excipients, buffer and antibody were more concentrated than in the liquid formulation (x1.43) to achieve 150mg lyophilized product per vial and no more than 1cm cake height. The formulations tested were:
30mg/ml anti-HER2 antibody, 5.76mM sodium acetate pH 5.7, 72mg/ml mannitol, 0.014% Tween80™; and 30mg/ml anti-HER2 antibody, 5.76mM sodium acetate pH 5.7, 72mg/ml mannitol, 0.043% Tween80™.

The lyophilization cycle performed is detailed in Table 7 below and also contained an annealing step to increase crystallinity.

**Table 7: Lyophilization cycle parameters**

| **Step** | **Temp (°C)** | **Vacuum (mbar)** | **Time (h)** |
|---|---|---|---|
| Freezing | 5 | | 0.15 |
| Freezing | 5 | | 0.30 |
| Freezing | -20 | | 0.25 |
| Freezing | -20 | | 0.3 |
| Freezing | -45 | | 0.35 |
| Freezing | -45 | | 2.00 |
| Freezing | -10 | | 1.15 |
| Freezing | -10 | | 2.00 |
| Freezing | -45 | | 1.15 |
| Freezing | -45 | | 2.00 |
| Vacuum | | 0.1 | |
| Primary drying | -30 | 0.1 | 1.00 |
| Primary drying | -30 | 0.1 | 72.00 |
| Secondary drying | 25 | | 5.00 |
| Secondary drying | 25 | | 24.00 |

*Results:* The starting product contained 99.8% monomers after purification. After the first test runs, the product contained 98.0% monomers (and 2.0% dimers) and no difference in the percentage of monomers or dimers was observed with either formulation. Therefore the different concentrations of Tween80™ did not affect the percentage of monomers in the lyophilized formulation. In conclusion, the first run shows that mannitol had a low cryoprotective effect (decrease of monomer content). A free-thaw study will therefore be performed before the next lyophilization runs to select excipients based on their cryoprotective capabilities. Given the minimal effect of Tween concentration (which is supposed to prevent the formation of aggregates during the reconstitution of the cake), it was decided to continue using the lowest Tween80™ concentration (0.014%), in this study.

### 2.2: Screen 2

*Aim:* In order to determine the optimal conditions between cake appearance and stability, different mixtures of mannitol and sucrose were tested over five freeze-thaw cycles.

*Materials* & *Method:* The formulations were prepared according to the General Materials & Methods section above. The formulations tested are shown in Table 9 below and were subjected to five freeze-thaw cycles at -40°C and 25°C. The formulations with the highest percentages of monomers were then selected for lyophilization using the lyophilization cycle as detailed in Table 7. Following lyophilization, the cakes were reconstituted and the percentage of monomers determined for each formulation.

*Results:* The results of five freeze-thaw cycles are shown in Figure 4. Formulations 6, 8, 10 and 19 were selected for lyophilisation since these formulations had the highest percentages of monomers present after freeze-thawing. After lyophilisation, formulation 10 showed the best cake appearance (results not shown). After reconstitution of the cakes, the percentage of monomers was determined and the results are shown in Figure 5. In conclusion, a combination of the lyoprotectants mannitol and sucrose imparted good stability on the lyophilized formulation of the anti-HER2 antibody; however the lyophilized formulations had a poor cake aspect. In order to test for improved formulation stability over these results, it was decided to test a higher concentration of sucrose. Furthermore, to try and improve the cake aspect, it was decided to test the addition of the bulking agent glycine to the formulation.

**Table 8: Mannitol/sucrose formulations subjected to five freeze-thaw cycles**

| No | Buffer | Mannitol % | Sucrose % | Tween80™ % |
|---|---|---|---|---|
| 1 | | 6 | 0 | 0.014 |
| 2 | | 4 | 0 | 0.014 |
| 3 | | 2 | 0 | 0.014 |
| 4 | | 0 | 4 | 0.014 |
| 5 | | 0 | 6 | 0.014 |
| 6 | | 1 | 6 | 0.014 |
| 7 | | 1 | 4 | 0.014 |
| 8 | | 2 | 6 | 0.014 |
| 9 | | 2 | 4 | 0.014 |
| 10 | 5.76mM sodium acetate pH 5.7 | 4 | 6 | 0.014 |
| 11 | | 4 | 4 | 0.014 |
| 12 | | 6 | 0 | 0.043 |
| 13 | | 4 | 0 | 0.043 |
| 14 | | 2 | 0 | 0.043 |
| 15 | | 0 | 4 | 0.043 |
| 16 | | 0 | 6 | 0.043 |
| 17 | | 1 | 6 | 0.043 |
| 18 | | 1 | 4 | 0.043 |
| 19 | | 2 | 6 | 0.043 |
| 20 | | 2 | 4 | 0.043 |
| 21 | | 4 | 6 | 0.043 |
| 22 | | 4 | 4 | 0.043 |

### 2.3: Screen 3

*Aim:* To determine the moisture and cake appearance of the preferred formulation comprising 4% mannitol and 6% sucrose from Screen 2 and also to test higher concentrations of both lyoprotectants to establish if cake aspect could be improved. In addition, the effect of a mixture of mannitol and glycine on the stability of the formulations was tested.

*Materials* & *Method:* The formulations were prepared according to the General Materials & Methods section above. The formulations tested are shown in Table 9 below and were subjected to lyophilization according to the parameters in Table 7.

**Table 9: Mannitol/sucrose/glycine formulations subjected to lyophilization**

| No | Buffer | Mannitol % | Sucrose % | Glycine mM | Tween80™ % |
|---|---|---|---|---|---|
| 1 | 5.76mM sodium acetate pH 5.7 | 4 | 6 | 0 | 0.014 |
| 2 | | 6 | 6 | 0 | 0.014 |
| 3 | | 0 | 6 | 0 | 0.014 |
| 4 | | 0 | 8 | 0 | 0.014 |
| 5 | | 0 | 10 | 0 | 0.014 |
| 6 | | 0 | 12 | 0 | 0.014 |
| 7 | | 5.6 | 0 | 3 | 0.014 |

*Results:* The percentage of moisture and cake appearance are described in Table 10 below for each formulation tested. Formulations 1, 2 and 7 showed good cake appearance; however formulation 2 had the highest moisture content. Formulations 3-6, which contained only sucrose had a shrunk and cracked cake appearance, although this was reduced at the higher sucrose concentrations of 10 and 12% (data not shown).

**Table 10: Moisture content and cake appearance of the seven formulations tested**

| No | Moisture % | Cake Appearance |
|---|---|---|
| 1 | 0.49 | Good, no shrinkage, no cracking |
| 2 | 0.80 | Good, no shrinkage, no cracking |
| 3 | 0.60 | Shrinkage and cracking |
| 4 | 0.59 | Shrinkage and cracking |
| 5 | 0.45 | Shrinkage and cracking |
| 6 | 0.55 | Shrinkage and cracking |
| 7 | 0.38 | Good, no shrinkage, no cracking |

### 2.4: Screen 4

*Aim:* The aim of this screen was to determine the stability of a formulation of the anti-HER2 antibody containing mannitol, glycine and Tween80™ as excipients. The formulations would first be subjected to five cycles of freeze-thaw and the most stable formulations selected for further lyophilization.

*Materials* & *Methods:* The formulations were prepared according to the General Materials & Methods section above. The formulations tested are shown in Table 11 below and were subjected to five freeze-thaw cycles at -40°C and 25°C.

**Table 11: Mannitol/glycine formulations subjected to five cycles of freeze-thaw**

| No | Buffer | Mannitol % | Glycine % | Tween80™ % |
|---|---|---|---|---|
| 1 | 5.76mM sodium acetate pH 5.7 | 2 | 0.022 | 0.014 |
| 2 | | 4 | 0.022 | 0.014 |
| 3 | | 6 | 0.022 | 0.014 |
| 4 | | 2 | 0.400 | 0.014 |
| 5 | | 4 | 0.400 | 0.014 |
| 6 | | 6 | 0.400 | 0.014 |
| 7 | | 2 | 0.600 | 0.014 |
| 8 | | 4 | 0.600 | 0.014 |
| 9 | | 6 | 0.600 | 0.014 |
| 10 | | 2 | 1.000 | 0.014 |
| 11 | | 4 | 1.000 | 0.014 |
| 12 | | 6 | 1.000 | 0.043 |

*Results:* The stability of the 12 formulations of the anti-HER2 antibody tested in five freeze-thaw cycles is shown in Figure 6, as the percentage of monomer present. It was observed during this study, that the ratio of mannitol:glycine appeared to be important for the stabilisation of the anti-HER2 antibody. Formulations 1, 4, 7, 10 and 11 were selected for further lyophilisation since the ratios of mannitol:glycine used had little effect on stability of the formulation before and after five cycles of freeze-thaw.

### 2.5: Screen 5

*Aim:* Since initial good stability results were observed with the mannitol/glycine formulations subjected to freeze-thaw cycles in Screen 4, it was decided to test a mixture of sucrose/glycine formulations and high sucrose concentration formulations alongside the selected mannitol/glycine formulations in a lyophilization cycle.

*Materials & Methods:* The formulations were prepared according to the General Materials & Methods section above. The formulations tested are shown in Table 12 below and were subjected to lyophilisation according to the parameters in Table 7.

**Table 12: Concentrations of the excipients tested in a lyophilization cycle in Screen 5**

| No | Buffer | Mannitol % | Sucrose % | Glycine % | Tween80™ % |
|---|---|---|---|---|---|
| 1 | | 0 | 10 | 0 | 0.014 |
| 2 | | 0 | 12 | 0 | 0.014 |
| | | | | | |
| 3 | | 0 | 10 | 0.4 | 0.014 |
| 4 | | 0 | 10 | 0.6 | 0.014 |
| 5 | | 0 | 10 | 1.0 | 0.014 |
| 6 | | 0 | 12 | 0.4 | 0.014 |
| 7 | | 0 | 12 | 0.6 | 0.014 |
| 8 | | 0 | 12 | 1.0 | 0.014 |
| | 5.76mM sodium acetate pH 5.7 | | | | |
| 9 | | 1 | 10 | 0 | 0.014 |
| 10 | | 2 | 10 | 0 | 0.014 |
| 11 | | 4 | 10 | 0 | 0.014 |
| 12 | | 1 | 12 | 0 | 0.014 |
| 13 | | 2 | 12 | 0 | 0.014 |
| 14 | | 4 | 12 | 0 | 0.014 |
| | | | | | |
| 15 | | 2 | 0 | 0.4 | 0.014 |
| 16 | | 2 | 0 | 0.6 | 0.014 |
| 17 | | 2 | 0 | 1.0 | 0.014 |
| 18 | | 4 | 0 | 1.0 | 0.014 |

*Results:* The stability of the 12 formulations of anti-HER2 antibody tested in five cycles of freeze-thaw is shown in Figure 7 as the percentage of monomer present. Cake appearance was also assessed (results not shown) and the seven formulations 4, 8, 10, 12, 15-17, which showed best stability and cake appearance, were selected for a one month stability study at 40°C.

### 2.5: Screen 6

*Aim:* To determine the stability of seven lyophilized anti-HER2 antibody formulations at 40°C for 1 month.

*Materials & Methods:* The formulations were prepared according to the General Materials & Methods section above. Seven formulations containing the excipients as listed in Table 13 were subjected to a lyophilization cycle according to the parameters as set out in Table 14.

**Table 13: Composition of the formulations subjected to lyophilization cycle**

| No | Buffer | Mannitol % | Sucrose % | Glycine % | Tween80™ % |
|---|---|---|---|---|---|
| 1 | 5.76mM sodium acetate pH 5.7 | 0 | 10 | 0.6 | 0.014 |
| 2 | | 0 | 12 | 1.0 | 0.014 |
| 3 | | 2 | 10 | 0.0 | 0.014 |
| 4 | | 1 | 12 | 0.0 | 0.014 |
| 5 | | 2 | 0 | 0.4 | 0.014 |
| 6 | | 2 | 0 | 0.6 | 0.014 |
| 7 | | 2 | 0 | 1.0 | 0.014 |

**Table 14: Lyophilization cycle parameters**

| **Step** | **Temp (°C)** | **Vacuum (mbar)** | **Time (h)** |
|---|---|---|---|
| Freezing | 5 | | 0.20 |
| Freezing | 5 | | 0.30 |
| Freezing | 0 | | 0.05 |
| Freezing | 0 | | 0.30 |
| Freezing | -45 | | 0.45 |
| Freezing | -45 | | 2.00 |
| Freezing/annealing | -15 | | 0.30 |
| Freezing/annealing | -15 | | 2.00 |
| Freezing | -45 | | 0.30 |
| Freezing | -45 | | 2.00 |
| Vacuum | | 0.1 | |
| Primary drying | -30 | 0.1 | 1.00 |
| Primary drying | -30 | 0.1 | 72.00 |
| Secondary drying | 25 | | 5.00 |
| Secondary drying | 25 | | 24.00 |

*Results:* The results of the stability study at 40°C for one month can be seen in Figures 8a, 8b and 8c. Formulations 1-4 showed a high percentage of monomers after one month (Figure 8a) as well as similar CEX profiles (Figure 8b). The percentage of moisture measured in the lyophilized cakes was shown to be lowest for formulations 1-4. One the basis of these results, formulations 2 and 4 were selected for use in further screens.

### 2.7: Screen 7

*Aim:* To optimise the lyophilization cycle and improve cake appearance by reducing the crack and shrinkage observed during secondary drying.

*Materials & Methods:* Formulations 2 and 4 were selected from Screen 6 and these were sent to Telstar (Terrassa, Spain) for determination of Collapse temperature (Tg').

The secondary drying step of the lyophilization cycle was examined to try and improve cake appearance and reduce the percentage of moisture in the cakes. Two new lyophilization protocols were tested in which the secondary drying step was slowed down. With reference to Table 14, the time for the two secondary drying steps was altered in one cycle from 5 and 24 hours to 15 and 9 hours, respectively and in a second cycle from 5 and 24 hours to 20 and 4 hours respectively, whilst a vacuum pressure of 0.1mbars was maintained throughout the secondary drying steps.

*Results:* The collapse temperatures of the two formulations tested are shown in Table 15 below, along with the composition of the formulations. Both formulations were considered to have very similar collapse temperatures.

**Table 15: Selected formulations and their Collapse temperature**

| No | Buffer | Mannitol % | Sucrose % | Glycine % | Tween80™ % | Tg' °C |
|---|---|---|---|---|---|---|
| 1 | 5.76mM sodium acetate pH 5.7 | 2 | 10 | 0 | 0.014 | -31 |
| 2 | | 0 | 12 | 1 | 0.014 | -32 |

Following lyophilization with a secondary drying step of 15 and 9 hours, the moisture content was determined to be 0.94% for formulation 1 and 1.19% for formulation 2. Following lyophilization with a secondary drying step of 20 and 4 hours, the moisture content was determined to be 1.20% for formulation 1 and 1.70% for formulation 2.

### 2.8: Screen 8

*Aim:* To determine the stability of the two selected anti-HER2 antibody lyophilized formulations at 40°C for one month. This study is the same as the one month stability study described in section 2.6 for Screen 6 but repeated due to the changes in the lyophilisation cycle described in section 2.7 above.

*Materials & Methods:* The composition of formulations 1 and 2 is shown in Table 16 below. Both formulations were subjected to the lyophilisation cycle shown in Table 17 below, where the secondary drying time was chosen to be 20 and 4 hours. The samples were analysed by SEC and CEX after one and two months storage at 40°C.

**Table 16: Composition of formulations 1 and 2**

| No | Buffer | Mannitol Sucrose % | Glycine % | Tween80™ % |
|---|---|---|---|---|
| 1 | 5.76mM sodium acetate pH 5.7 | 2 10 | 0 | 0.014 |
| 2 | | 0 12 | 1 | 0.014 |

**Table 17: Optimized lyophilisation cycle parameters**

| **Step** | **Temp (°C)** | **Vacuum (mbar)** | **Time (h)** |
|---|---|---|---|
| Freezing | 5 | | 0.20 |
| Freezing | 5 | | 0.30 |
| Freezing | 0 | | 0.05 |
| Freezing | 0 | | 0.30 |
| Freezing | -45 | | 0.45 |
| Freezing | -45 | | 2.00 |
| Freezing/annealing | -15 | | 0.30 |
| Freezing/annealing | -15 | | 2.00 |
| Freezing | -45 | | 0.30 |
| Freezing | -45 | | 2.00 |
| Vacuum | | 0.1 | |
| Primary drying | -30 | 0.1 | 1.00 |
| Primary drying | -30 | 0.1 | 72.00 |
| Secondary drying | 25 | 0.1 | 20.00 |
| Secondary drying | 25 | 0.1 | 4.00 |

*Results:* The percentage of monomers in both candidate formulations after one and two months as measured by HPLC-SEC can be seen in Figure 9a. The HPLC-CEX results are shown in Figure 9b. At time 0, for the liquid and lyophilized formulations, there is little difference in stability for Formulations 1 and 2. After one month storage at 40°C, there is no difference between the formulations following HPLC-CEX; however there is a small difference shown by HPLC-SEC in Figure 9a. It can be clearly seen from both Figures 9a and 9b that after two months storage, Formulation 2 is more stable than Formulation 1. Formulation 2 was then chosen to be taken forward for use in a long term stability study.

### Example 3 - Long term stability study

*Aim:* The purpose of this stability study was to determine the stability of the lyophilized anti-HER2 antibody formulation at a target concentration of 30 g/L, for intended storage at 5 ± 3°C, an accelerated study at 25 ± 2°C and a stress study 40 ± 2°C. The stability of the lyophilized anti-HER2 antibody formulation will be followed for 36 months at 5 ± 3°C, 12 months at 25 ± 2°C and 3 months at 40 ± 2°C.

*Materials* & *Method:* The formulation buffer was 5.75mM Sodium Acetate pH 5.7 (Merck), 12% Sucrose (Sigma), 1% glycine (Merck) and 0.014% Tween80™ (VWR). Hydrochloric Acid 37% (Sigma) was used for pH adjustment. Specifications for the buffer were 5.7 ± 0.1 for the pH and 0.42 to 0.66mS/cm for the conductivity. All chemicals were suitable for parenteral administration. The buffer was then filtered through a 0.22µm filter into a bag.

Stock solutions of the three families of excipients, lyoprotectant (sucrose), bulking agent (glycine) and surfactant (Tween80™) were prepared in 5.75mM sodium acetate buffer pH 5.7. Stock solutions of sucrose, glycine and Tween80™ were prepared at concentrations of 600g/L, 130 g/L and 100g/L, respectively. The excipients were then added to the anti-HER2 antibody formulation by dilution of the stock solution in the formulation to achieve the desired concentration.

Purified anti-HER2 antibody was diafiltrated 7-10 times by tangential flow filtration (TFF) in 5.75mM sodium acetate pH 5.7 and then concentrated to 50-55g/L. After TFF, the anti-HER2 antibody was formulated by the addition of sucrose, glycine and Tween80™ to reach the final buffer composition. The reference standard used was DRS-anti-HER2 antibody-01 used for the analytical testing and kept under Quality Control at a temperature of below -60°C.

To prepare the lyophilized drug product, a Telstar lyophilization system was used (Lyobeta 15 lyophilizer; Telstar, Terrassa, Spain). A VP600 Vötsch stability chamber (Vötsch Industrietechnik GmbH, Germany) is used for the 3 months study at 40 ± 2°C, ± 5% relative humidity test and also for the 12 months study at 25 ± 2°C, ± 5% relative humidity test. A standard laboratory fridge is used for the 36 months stability at 5 ± 3°C.

Vials and stopper materials used were those intended for clinical trial manufacture. Vials were 20 ml Fiolax clear, USP type I glass vials purchased from Schott (Art. No: 1156521) and the stoppers were Flurotec distributed by West Pharma (ref FD20TT3WRS). All vials were crimped.

The formulated anti-HER2 antibody was aliquotted in 20ml Schott vials at a volume of 5ml per vial. A total of 62 vials were prepared. The rest of the shelf was filled with placebo vials. The vials were lyophilized according to the parameters in Table 18 below.

**Table 18: Lyophilisation cycle parameters for the long term stability study**

| **Step** | **Temp (°C)** | **Vacuum (mbar)** | **Time (h)** |
|---|---|---|---|
| Freezing | 5 | | 0.20 |
| Freezing | 5 | | 0.30 |
| Freezing | 0 | | 0.05 |
| Freezing | 0 | | 0.30 |
| Freezing | -45 | | 0.45 |
| Freezing | -45 | | 2.00 |
| Freezing/annealing | -15 | | 0.30 |
| Freezing/annealing | -15 | | 2.00 |
| Freezing | -45 | | 0.30 |
| Freezing | -45 | | 2.00 |
| Vacuum | | 0.1 | |
| Primary drying | -30 | 0.1 | 1.00 |
| Primary drying | -30 | 0.1 | 72.00 |
| Secondary drying | 25 | 0.1 | 20.00 |
| Secondary drying | 25 | 0.1 | 4.00 |

Stability of the drug product was determined using a number of tests to evaluate changes in the drug product over time. CEX-HPLC (Dionax, ProPac WCX-104 x 250mm), SEC-HPLC (Phenomenex, YARRA 3u SEC-3000 7.8 x 300 mm) and SDS-PAGE are sensitive indicators of product breakdown. The profile of drug product using these assays will change over time if the sample is subject to degradation. The A280 assay can be used to detect changes in the protein level which may be indicative of product breakdown or adhesion to the container wall. An ELISA cell based assay and an ELISA binding assay can be used to determine the potency of the product. A change in the potency of the sample with time is indicative of product deterioration. The pH of the formulation will also be checked over time to determine pH stability and the physical appearance of the solution also monitored. The presence or absence of sub visible particles after dissolution of the cake will also be checked. Any variation in moisture of the product will be monitored. This can increase the sensitivity of the lyophilized product to degradation and will be checked by a Karl Fisher titration. Finally, reconstitution time will be measured and used to determine the complete dissolution time of the lyophilized product cakes. These parameters will be assessed according to the criteria shown in Table 19 below for the time points for the temperatures 5°C, 25°C and 40°C.

**Table 19: Parameters for assessing stability of the anti-HER2 antibody drug product**

| **Parameter** | **Specifications** |
|---|---|
| SEC - HPLC | monomer > 98% |
| CEX-HPLC | comparable to reference standard* |
| SDS-PAGE | comparable to reference standard* |
| A280 | ≥20 and ≤24 mg/ml |
| ELISA Binding | ≥75% and ≤125% of EC₅₀ of reference standard* |
| pH | ≥ 5.6 and ≤ 5.8 |
| Appearance | Colorless, clear to slightly opalescent liquid Practically free of visible particulates |
| Reconstitution Time | ≤ 3 min |
| Sub visible particles | ≤ 6000 particles ≥10µm/vials ≤ 600 particles ≥ 25µm/vials |
| Karl Fisher titration | ≤ 2% |

| | |
|---|---|
| * Reference standard used was DRS-anti-HER2 antibody-01 | |

*Results:* The percentage of monomers in the lyophilized anti-HER2 antibody formulation after 12 months as measured by HPLC-SEC is shown in Figure 10a. This figure shows that there is no difference in percentage monomer in the formulation samples at all time points and at all temperatures tested. The HPLC-CEX results for 12 months are shown in Figure 10b. The large scale used on the y-axis of the graph gives the impression of a large difference between the stability of the formulation samples, particularly at 40°C and later time points; however there is actually only a difference at the most of approximately 1.5% and the formulation was considered stable for the duration of the 12 month study. The results for the other parameters tested are summarised in Table 20 below where it is indicated whether the specification for each assessment parameter was met.

For most of the parameters tested, the anti-HER2 antibody formulation tested was found to be within the specifications as given in Table 19, for all temperatures and time points tested up to and including 12 months. For the A280 assay, used to detect changes in the protein level which may be indicative of product breakdown or adhesion to the container wall, the initial assessment specification of ≥20 and ≤24 mg/ml was miscalculated. At the start of the stability study the result for the A280 assay was actually 18 mg/ml (already outside of the assessment specification) and this figure remained the same throughout the study, indicating that there was actually no product breakdown or adhesion to the container wall. For the assessment of pH, the specification of a pH of ≥ 5.6 and ≤ 5.8 were set. For the readings at the 6 month time point at 5°C and 25°C, the pH values recorded were 5.865, only fractionally outside of the pH range set. At all other time points and temperatures tested the pH specifications were within the range set.

In conclusion, this stability study over 12 month has demonstrated that the anti-HER2 antibody formulation of the present invention has met the majority of the assessment parameters for stability and is therefore expected to be stable for the remaining duration of the long term stability study.

**Table 20: Summary of stability assessment parameters for the anti-HER2 antibody drug product at the times and temperatures tested**

| **Month** | **0 liquid** | **0 after lyo** | **1** | | | **2** | | | **3** | | | **6** | | **9** | | **12** | |
|---|---|---|---|---|---|---|---|---|---|---|---|---|---|---|---|---|---|
| **Temp °C** | | | **5** | **25** | **40** | **5** | **25** | **40** | **5** | **25** | **40** | **5** | **25** | **5** | **25** | **5** | **25** |
| **Parameter** | | | | | | | | | | | | | | | | | |
| SEC - HPLC | ✔ | ✔ | ✔ | ✔ | ✔ | ✔ | ✔ | ✔ | ✔ | ✔ | ✔ | ✔ | ✔ | ✔ | ✔ | ✔ | ✔ |
| CEX-HPLC | ✔ | ✔ | ✔ | ✔ | ✔ | ✔ | ✔ | ✔ | ✔ | ✔ | ✔ | ✔ | ✔ | ✔ | ✔ | ✔ | ✔ |
| SDS-PAGE | ✔ | ✔ | ✔ | ✔ | ✔ | ✔ | ✔ | ✔ | ✔ | ✔ | ✔ | ✔ | ✔ | ✔ | ✔ | ✔ | ✔ |
| A280 | 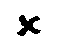 | 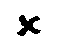 | 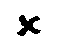 | 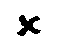 | 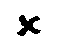 | 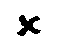 | 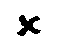 | 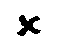 | 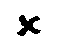 | 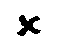 | 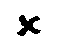 | 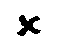 | 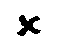 | 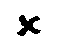 | 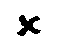 | 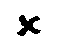 | 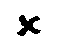 |
| ELISA Binding | 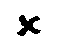 | ✔ | ✔ | ✔ | 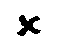 | ✔ | ✔ | ✔ | ✔ | ✔ | ✔ | ✔ | ✔ | ✔ | ✔ | | |
| pH | ✔ | ✔ | ✔ | ✔ | ✔ | ✔ | ✔ | ✔ | ✔ | ✔ | ✔ | 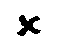 | 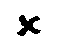 | ✔ | ✔ | ✔ | ✔ |
| Appearance | ✔ | ✔ | ✔ | ✔ | ✔ | ✔ | ✔ | ✔ | ✔ | ✔ | ✔ | ✔ | ✔ | ✔ | ✔ | ✔ | ✔ |
| Reconstitution Time | N/A | N/A | ✔ | ✔ | ✔ | ✔ | ✔ | ✔ | ✔ | ✔ | ✔ | ✔ | ✔ | ✔ | ✔ | ✔ | ✔ |
| Sub visible particles | nd | ✔ | nd | nd | nd | nd | nd | nd | nd | nd | nd | nd | nd | nd | nd | ✔ | ✔ |
| Karl Fisher titration | nd | ✔ | nd | nd | nd | nd | nd | nd | nd | nd | ✔ | nd | nd | nd | nd | ✔ | ✔ |

| | | | | | | | | | | | | | | | | | |
|---|---|---|---|---|---|---|---|---|---|---|---|---|---|---|---|---|---|
| ✔: parameter met; 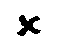 : parameter unmet; nd: not determined; N/A: not applicable | | | | | | | | | | | | | | | | | |

## Claims

1. A pharmaceutical formulation comprising an anti-HER2 antibody or a fragment thereof that recognizes and binds to human HER2 and which has a heavy chain of SEQ ID NO: 10 and a light chain of SEQ ID NO: 11; an acetate buffer, wherein the acetate buffer is sodium acetate buffer present within the pharmaceutical formulation in an amount of between 1 and 10mM; a surfactant, wherein the surfactant is Polysorbate 80, present within said pharmaceutical formulation in an amount of between 0.001% and 0.1% (w/w); a lyoprotectant, wherein the lyoprotectant is sucrose present within the pharmaceutical formulation in an amount of between 5% and 15% (w/w) or sucrose and mannitol present within the pharmaceutical formulation in an amount of between 5% and 15% (w/w) sucrose and 0.5% and 5% (w/w) mannitol; and a bulking agent, wherein the bulking agent is glycine present within the pharmaceutical formulation in an amount of between 0.5% and 1.5%(w/w) and wherein the pH of the formulation is between 5.9 and 6.0

2. The pharmaceutical formulation according to claim 1, wherein the pharmaceutical formulation is a liquid formulation, a lyophilized formulation or a reconstituted formulation.

3. The pharmaceutical formulation according to claim 1 or claim 2, wherein the anti-HER2 antibody or fragment thereof that recognizes and binds to human HER2, is present within the pharmaceutical formulation in an amount of between 10 and 40 mg/ml.

4. A lyophilized pharmaceutical formulation according to claim 1, which comprises 30mg/ml of an anti-HER2 antibody or a fragment thereof that recognizes and binds to human HER2, 12% (w/w) sucrose, 1% (w/w) glycine and 0.014% (w/w) Polysorbate 80 and 5.75mM sodium acetate buffer, wherein the pH of the formulation is 5.7 ± 0.2.

5. The pharmaceutical formulation according to claim 4, wherein the formulation comprises a diluent and is a reconstituted formulation.

6. The pharmaceutical formulation according to claim 5, which comprises 21mg/ml of an anti-HER2 antibody or a fragment thereof that recognizes and binds to human HER2, 8.4% (w/w) sucrose, 0.7% (w/w) glycine, 0.01% (w/w) Polysorbate 80, WFI and 4mM sodium acetate, wherein the pH of the formulation is 5.7 ± 0.2.

7. The pharmaceutical formulation according to any one of the preceding claims for use in the treatment of a HER2 related disorder selected from a HER2 positive cancer such as metastatic breast cancer, early breast cancer and metastatic gastric cancer.

8. An article of manufacture comprising:
(a) a container which holds a lyophilized formulation according to any one of claims 4-7; and
(b) instructions for reconstituting the lyophilized formulation with a diluent to an antibody concentration in the reconstituted formulation of about 10 to 40mg/ml.

## Patentansprüche

1. Pharmazeutische Formulierung, umfassend einen Anti-HER2-Antikörper oder ein Fragment davon, der menschliches HER2 erkennt und daran anbindet und das eine schwere Kette der SEQ ID NO: 10 und eine leichte Kette der SEQ ID NO: 11 aufweist; einen Acetatpuffer, wobei der Acetatpuffer ein Natriumacetatpuffer ist, der in der pharmazeutischen Formulierung in einer Menge von zwischen 1 bis 1O mM vorhanden ist; ein Tensid, wobei das Tensid Polysorbat 80 ist, das in der pharmazeutischen Formulierung in einer Menge von zwischen 0,001% und 0,1% (w/w) vorhanden ist;
ein Lyoprotektionsmittel, wobei das Lyoprotektionsmittel Saccharose ist, die in der pharmazeutischen Formulierung in einer Menge von zwischen 5% und 15% (w/w) vorhanden ist, oder Saccharose und Mannitol, die in der pharmazeutischen Formulierung in einer Menge zwischen 5% und 15% (w/w) Saccharose und 0,5% und 5% (w/w) Mannitol vorhanden sind;
und ein Füllmittel, wobei
das Füllmittel Glycin ist, das in der pharmazeutischen Formulierung in einer Menge von 0,5% bis 1,5% (w/w) vorhanden ist, und wobei der pH-Wert der Formulierung zwischen 5,9 und 6,0 liegt.

2. Pharmazeutische Formulierung nach Anspruch 1, wobei die pharmazeutische Formulierung eine flüssige Formulierung, eine lyophilisierte Formulierung oder eine wiederhergestellte Formulierung ist.

3. Pharmazeutische Formulierung nach Anspruch 1 oder 2, wobei der Anti-HER2-Antikörper oder ein Fragment davon, der menschliches HER2 erkennt und daran anbindet, in der pharmazeutischen Formulierung in einer Menge von zwischen 10 und 40 mg/ml vorhanden ist.

4. Lyophilisierte pharmazeutische Formulierung nach Anspruch 1, die 30 mg/ml eines Anti-HER2-Antikörpers oder eines Fragments davon, das menschliches HER2 erkennt und daran anbindet, 12% (w/w) Saccharose, 1% (w/w) Glycin und 0,014% (w/w) Polysorbat 80 und 5,75 mM Natriumacetatpuffer umfasst, wobei der pH-Wert der Formulierung 5,7 ± 0,2 beträgt.

5. Pharmazeutische Formulierung nach Anspruch 4, wobei die Formulierung ein Verdünnungsmittel umfasst und eine wiederhergestellte Formulierung ist.

6. Pharmazeutische Formulierung nach Anspruch 5, die 21 mg/ml eines Anti-HER2-Antikörpers oder eines Fragments davon, das menschliches HER2 erkennt und daran anbindet, 8,4% (w/w) Saccharose, 0,7% (w/w) Glycin, 0,01% (w/w) Polysorbat 80, WFI und 4 mM Natriumacetat umfasst, wobei der pH-Wert der Formulierung 5,7 ± 0,2 beträgt.

7. Pharmazeutische Formulierung nach einem der vorhergehenden Ansprüche zur Verwendung bei der Behandlung einer HER2-bezogenen Erkrankung, ausgewählt aus einem HER2-positiven Krebs, wie metastasiertem Brustkrebs, Brustkrebs im Frühstadium und metastasierendem Magenkrebs.

8. Erzeugnis, umfassend:
(a) einen Behälter, der eine lyophilisierte Formulierung nach einem der Ansprüche 4-7 enthält; und
(b) Anweisungen zum Wiederherstellen der lyophilisierten Formulierung mit einem Verdünnungsmittel zu einer Antikörperkonzentration in der wiederhergestellten Formulierung von etwa 10 bis 40 mg/ml.

## Revendications

1. Formulation pharmaceutique comprenant un anticorps anti-HER2 ou un fragment de celui-ci qui reconnaît et se lie à HER2 humain et qui a une chaîne lourde de SEQ ID NO : 10 et une chaîne légère de SEQ ID NO : 11 ; un tampon acétate, le tampon acétate étant le tampon acétate de sodium présent dans la formulation pharmaceutique dans une quantité d'entre 1 et 10 mM ; un tensio-actif, le tensio-actif étant le Polysorbate 80, présent dans ladite formulation pharmaceutique dans une quantité d'entre 0,001 % et 0,1 % (p/p) ; un lyoprotecteur, le lyoprotecteur étant le sucrose présent dans la formulation pharmaceutique dans une quantité d'entre 5 % et 15 % (p/p) ou le sucrose et le mannitol présents dans la formulation pharmaceutique dans une quantité d'entre 5 % et 15 % (p/p) de sucrose et 0,5 % et 5 % (p/p) de mannitol ; et un agent gonflant, l'agent gonflant étant la glycine présente dans la formulation pharmaceutique dans une quantité d'entre 0,5 % et 1,5 % (p/p) et le pH de la formulation étant entre 5,9 et 6,0.

2. Formulation pharmaceutique selon la revendication 1, dans laquelle la formulation pharmaceutique est une formulation liquide, une formulation lyophilisée ou une formulation reconstituée.

3. Formulation pharmaceutique selon la revendication 1 ou la revendication 2, dans laquelle l'anticorps anti-HER2 ou le fragment de celui-ci qui reconnaît et se lie à HER2 humain, est présent dans la formulation pharmaceutique dans une quantité d'entre 10 et 40 mg/ml.

4. Formulation pharmaceutique lyophilisée selon la revendication 1, qui comprend 30 mg/ml d'un anticorps anti-HER2 ou d'un fragment de celui-ci qui reconnaît et se lie à HER2 humain, 12 % (p/p) de sucrose, 1 % (p/p) de glycine et 0,014 % (p/p) de Polysorbate 80 et un tampon d'acétate de sodium 5,75 mM, le pH de la formulation étant de 5,7 ± 0,2.

5. Formulation pharmaceutique selon la revendication 4, dans laquelle la formulation comprend un diluant et est une formulation reconstituée.

6. Formulation pharmaceutique selon la revendication 5, qui comprend 21 mg/ml d'un anticorps anti-HER2 ou d'un fragment de celui-ci qui reconnaît et se lie à HER2 humain, 8,4 % (p/p) de sucrose, 0,7 % (p/p) de glycine, 0,01 % (p/p) de Polysorbate 80, eau pour injection (WFI) et acétate de sodium 4 mM, le pH de la formulation étant de 5,7 ± 0,2.

7. Formulation pharmaceutique selon l'une quelconque des revendications précédentes pour une utilisation dans le traitement d'un trouble se rapportant à HER2 choisi parmi un cancer positif à HER2 tel que le cancer du sein métastatique, le cancer du sein précoce et le cancer gastrique métastatique.

8. Article de fabrication comprenant :
(a) un récipient qui contient une formulation lyophilisée selon l'une quelconque des revendications 4 à 7, et
(b) des instructions pour reconstituer la formulation lyophilisée avec un diluant à une concentration d'anticorps dans la formulation reconstituée d'environ 10 à 40 mg/ml.


### SEQUENCE LISTING

<110> Glenmark Pharmaceuticals S.A.
<120> Antibody formulation
<130> PCT application
<150> US 61/745,293
   <151> 2012-12-21
<160> 11
<170> PatentIn version 3.5
<210> 1
   <211> 1255
   <212> PRT
   <213> Homo sapiens
<400> 1
<210> 2
   <211> 10
   <212> PRT
   <213> artificial
<220>
   <223> anti-HER2 antibody HCDR1
<400> 2
   Gly Phe Asn Ile Lys Asp Thr Tyr Ile His
   1               5                   10
<210> 3
   <211> 17
   <212> PRT
   <213> Artificial
<220>
   <223> anti-HER2 antibody HCDR2
<400> 3
   Arg Ile Tyr Pro Thr Asn Gly Tyr Thr Arg Tyr Ala Asp Ser Val Lys
   1               5                   10                  15
   Gly
<210> 4
   <211> 11
   <212> PRT
   <213> Artificial
<220>
   <223> anti-HER2 antibody HCDR3
<400> 4
   Trp Gly Gly Asp Gly Phe Tyr Ala Met Asp Tyr
   1               5                   10
<210> 5
   <211> 11
   <212> PRT
   <213> Artificial
<220>
   <223> anti-HER2 antibody LCDR1
<400> 5
   Arg Ala Ser Gln Asp Val Asn Thr Ala Val Ala
   1               5                   10
<210> 6
   <211> 7
   <212> PRT
   <213> Artificial
<220>
   <223> anti-HER2 antibody LCDR2
<400> 6
   Ser Ala Ser Phe Leu Tyr Ser
   1               5
<210> 7
   <211> 9
   <212> PRT
   <213> Artificial
<220>
   <223> anti-HER2 antibody LCDR3
<400> 7
   Gln Gln His Tyr Thr Thr Pro Pro Thr
   1               5
<210> 8
   <211> 120
   <212> PRT
   <213> Artificial
<220>
   <223> anti-HER2 antibody VH
<400> 8
<210> 9
   <211> 107
   <212> PRT
   <213> Artificial
<220>
   <223> anti-HER2 antibody VL
<400> 9
<210> 10
   <211> 450
   <212> PRT
   <213> Artificial
<220>
   <223> anti-HER2 antibody Heavy chain
<400> 10
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
   450
<210> 11
   <211> 214
   <212> PRT
   <213> Artificial
<220>
   <223> anti-HER2 antibody Light chain
<400> 11
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Asn Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Arg Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210